(19) Europäisches Patentamt
European Patent Office
Office européen des brevets

(11) Publication number: 0 244 688 B1

(12) EUROPEAN PATENT SPECIFICATION

(45) Date of publication of patent specification: 23.10.91 Bulletin 91/43

(51) Int. Cl.[5]: C09J 201/00, C09D 201/00, A61L 25/00, A61L 27/00

(21) Application number: 87105775.8

(22) Date of filing: 18.04.87

# (54) Adhesives derived from bioadhesive polyphenolic proteins.

(30) Priority: 25.04.86 US 856597
02.04.87 US 34078

(43) Date of publication of application:
11.11.87 Bulletin 87/46

(45) Publication of the grant of the patent:
23.10.91 Bulletin 91/43

(84) Designated Contracting States:
AT BE CH DE ES FR GB GR IT LI LU NL SE

(56) References cited:
US-A- 3 438 374
US-A- 4 585 585
SCIENCE, vol. 212, 29th May 1981, pages 1038-1040, AAAS; J.H. WAITE et al.: "Polyphenolic substance of Mytilus edulis: novel adhesive containing L-dopa and hydroxyproline"

(56) References cited:
CHEMICAL ABSTRACTS, vol. 94, 1981, page 254, no. 152234h, Columbus, Ohio, US; E. LINDNER: "Experiments in synthesis of barnacle adhesive" & BIOL. MAR., COMMUN., CONGR. INT. CORROS. MAR. INCRUSTACIONES, 5TH 1980, 189-212
BIOCHEMICAL AND BIOPHYSICAL RESEARCH COMMUNICATIONS, vol. 96, no. 4, 31st October 1980, pages 1554-1561, Academic Press Inc.; J.H. WAITE et al.: "The bioadhesive of Mytilus byssus: a protein containing L-dopa"

(73) Proprietor: Bio-Polymers, Inc.
309 Farmington Avenue
Farmington Connecticut 06032 (US)

(72) Inventor: Benedict, Christine V.
36 Gail Road
Farmington Connecticut 06032 (US)
Inventor: Picciano, Paul T.
31 Colony Road
Canton Connecticut 06109 (US)

(74) Representative: Hoeger, Stellrecht & Partner
Uhlandstrasse 14 c
W-7000 Stuttgart 1 (DE)

EP 0 244 688 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

CROSS REFERENCE

This application is a continuation-in part of copending application Serial No. 856,597 filed April 25, 1986.

BACKGROUND OF THE INVENTION

This invention relates to adhesive formulations derived from bioadhesive polyphenolic proteins which are useful in biomedical applications and which are particularly well suited for use in aqueous environments. Bioadhesive polyphenolic proteins, originally derived from several species of the mussel genus Mytilus, can be derived either from natural sources or be manufactured synthetically, and contain one or more sequences of repeating decapeptides having the formula:

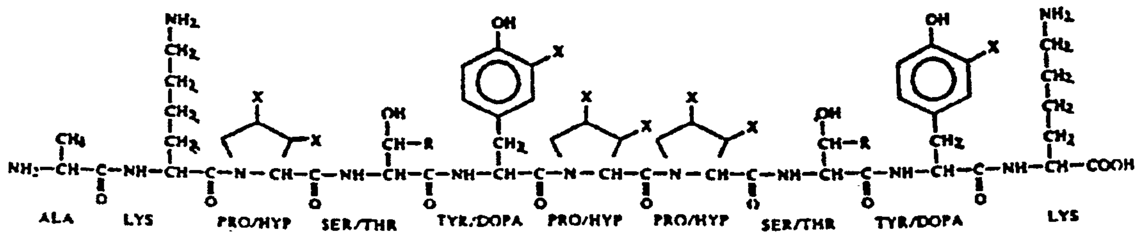

wherein each X is independently selected from the group comprising hydroxyl and hydrogen; and wherein each R is independently selected from the group comprising hydrogen and methyl. As used in this application, the term "bioadhesive polyphenolic proteins" is to be understood as referring to mixtures of proteins containing from 1 to about 1,000 units of the above repeating decapeptides, and optionally may contain other proteinaceous units, and chain extenders.

Naturally-occurring protein from which the bioadhesive polyphenolic protein may be derived is produced and stored in the exocrine phenol gland of the mussel and is deposited onto marine surfaces by the mussel's foot during the formation of new adhesive plaques. Decapeptides may be obtained from the bioadhesive polyphenolic proteins by the method described by Waite in Journal of Biological Chemistry 258, 2911-15 (1983), and in United States Patent No. 4,585,585 (intermediate document).

J.H. Waite and M.L. Tanzer report in Science, vol. 212 (1981), 1038-1040, on the amino acid content found in the polyphenolic protein of the penol gland of Mytilus edulis, especially the large amounts of lysine, DOPA, and 3- and 4-hydroxyproline. However, this reference falls short in teaching how the polyphenolic protein may be processed and formulated in order to obtain an excellent adhesive on the basis of this naturally occurring protein.

The same authors already reported on previous results in Biochemical and Biophysical Research Communications, vol. 96 (1980), 1554-1561. Unusually high contents of lysine and DOPA are mentioned and the ability of DOPA quinoid residues to cross-link with lysyl residues. Such a cross-linking is assumed to take place in thread formation. The tread, however, is not responsable for the adhesional effect. For the adhesional disc of the mussels it is only stated that the content of the cross-linkable amino acids lysine and DOPA is very much reduced when compared to their presence in the polyphenolic protein.

Bioadhesive polyphenolic proteins exhibit excellent adhesive properties on a variety of surfaces, particularly surfaces submerged in water. The repeating decapeptides of the bioadhesive polyphenolic protein, are in essence the building blocks for a potentially wide variety of adhesive and coating substances.

The decapeptides and the bioadhesive polyphenolic proteins containing them show great promise for the development of commercial products. Virtually all priot art adhesives perform optimally when first applied on clean, dry surfaces. However, even those adhesives which display water resistant characteristics after curing, for example, resorcinol-formaldehyde polymers, fail if applied in excessively moist environments. Bioadhesive polyphenolic proteins and their constituent decapeptides, on the other hand, have the potential to impart water-compatible characteristics to any adhesive formulation through their increased monomeric molecular weight, reduced tendency to diffuse from the application site, and increased number and variety of reactive residues, such as the "phenol-like" residues tryosine and dopa, that are especially capable of displacing water.

However, to date no compositions containing bioadhesive polyphenolic proteins have been available for scientific, medical or commercial use.

Accordingly, it is a principal object of this invention to provide compositions derived from bioadhesive polyphenolic proteins for use in various applications involving the need for adhesion in at least a partially aqueous environment, which applications specifically include, but are not limited to, medicine and surgery, botany, dentistry, underwater applications, chromatography, and the like.

## SUMMARY OF THE INVENTION

The adhesive formulations of this invention comprise by weight: (1) from about 5% to about 99% of bioadhesive polyphenolic proteins which contain repeating decapeptides having the formula:

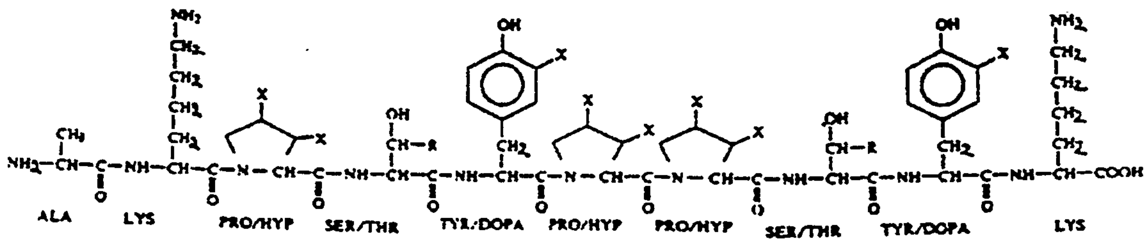


wherein each X is independently hydrogen or hydroxyl, and wherein each R is independently hydrogen or methyl; (2) from about 1.0% to about 40% of a cross-linking agent to promote at least partial cross-linking of the bioadhesive polyphenolic proteins; (3) from about 0% to about 90% of one or more additives for providing desired properties in the completed adhesive/coating formulation; and (4) from about 0 to about 50% of one or more filling agents compatible with the substrate to which the composition is to be adhered.

## DETAILED DISCLOSURE

Adhesive formulations of this invention comprise bioadhesive polyphenolic proteins which contain repeating decapeptides, cross-linking agents which promote at least partial cross-linking of the bioadhesive polyphenolic proteins, optionally, one or more additives for providing desired properties in the completed adhesive formulation, and optionally, one or more filling agents compatible with the substrate to which the composition is to be adhered. For the purposes of this description, it should be understood that adhesive formulations may be used to bind two substrates together (a true adhesive application) and in applications wherein the adhesive formulation is applied as a thin film on a substrate (a coating application). In this coating application, bioadhesive polyphenolic protein formulations are useful, for example, in marine environments to prevent the fouling of certain substrates coated with a film of the formulation when submerged for long periods of time under sea or fresh water.

Bioadhesive polyphenolic proteins consist in large part of the above-depicted repeating decapeptide sequence, present from 1 to about 1,000 times. Bioadhesive polyphenolic proteins thus comprise repeating decapeptide sequences of varying lengths and may also comprise other proteins and chain linking groups such as amino acids, oligopeptides, and various other bifunctional spacers. Amino acids comprise any of the well-known naturally-occurring L-amino acids, as well as other amino acids such as ornithine, homo-cysteine, citrulline, 3-aminotyrosine, and the like. Oligopeptides include the various di-, tri, tetra-, or penta-peptides and higher peptides which can be readily synthesized or are commercially available. Examples include (ALA-CYS-ALA), $(ALA\text{-}LYS)_3$, $(ALA\text{-}LYS\text{-}PRO)_4$, $(PRO\text{-}HYP\text{-}GLY)_5$ and the like. Bifunctional spacers include such diverse substances as aliphatic or aromatic dialdehydes, imido esters, isocyanates, aryl and alkyl dihalides, dimaleimides, and the like. Bifunctional spacers also include all the chemical mediators of peptide bond formations, anhydrides, active esters, condensing agents (dicyclohexylcarbodiimide) used in solid and liquid phase peptide synthesis.

The concentration of bioadhesive polyphenolic proteins in the adhesive compositions may vary from about 5% by weight to about 99% by weight, the exact concentration selected depending upon the intended use of the composition.

The second component of the adhesive composition is a cross-linking agent which may be used to promote partial or full cross-linking of the bioadhesive polyphenolic proteins between substrates and the proteins and/or between the additives in the formultion and the proteins. The nature of the cross-linking is uncertain, but is believed to involve covalent bonds, ionic bonds, hydrogen bonds, and Van der Waals bonds, or a combination of these bonds. Here again, the amount of cross-linking agent utilized will depend upon the ultimate use of the

adhesive formulation and can vary in amount ranging from about 1.0% to about 40% by weight (5,000 to 50,000 Units/mg adhesive proteins by activity) or more based on the number of repeating decapeptide sequences in the bioadhesive polyphenolic proteins. The precise weight percent of cross-linker used, of course, depends upon the molecular weight of the bioadhesive polyphenolic protein and the purity of the cross-linking agent. Suitable cross-linking agents include, for example, enzymatic oxidizing agents such as catechol oxidase, mushroom tyrosinase, or chemical cross-linking agents with any number of reactive functional groups, such agents including glutaraldehyde, formaldehyde, or even chemical oxidizing agents such as oxygen or a peroxide.

The third component of the adhesive formulation is one or more additives which may be used to promote the desired properties in the adhesive composition. For almost all applications, a cationic, anionic or non-ionic surfactant additive may be used, the choice of which depends on the intended use of the particular formulation, and which selection is well within the knowledge of persons skilled in the art. Examples of such surfactants are sodium dodecylsulfate, a sulfate-rich compound, protein or mucoprotein, and sodium dodecylbenzenesulfonate. Other additives may be used, depending on the desired use of the composition. For example, if the adhesive formulation is intended to be conductive for use with two electrically conductive substrates, conductive additives such as a metal salt, for example, silver chloride, silver nitrate, ferric chloride, cuprous sulfate, cupric sulfate, or organometallic coordination compounds such as ferrocene or the like may be used. These additives may comprise up to about 90% by weight of the adhesive composition.

The fourth component of the adhesive composition is one or more fillers, the nature of which depends primarily on the composition and surface characteristics of the substrate to be bonded or sealed. For example, in biomedical applications, the filling agent can be a substance having biocompatible properties such as collagen, albumin, hyaluronic acid, hydroxyapatite, chondroitan sulfate, elastin, laminin, casein, chitin, chitosan, or similar substances. Alternatively (or in addition to the foregoing filling agent), the filling agent may be an inert material similar in nature to one or both of the substrates being joined, as, for example, silica beads for glass, ceramic, or cement substrates, wood or cellulosic fibers or other polysaccharides for woody or non-woody plant tissue, and synthetic polyesters, polyurethanes, polyamines, polyacetates, and the like for induatrial (metallic or non-metallic) substrates. The percentage of filler utilized may be up to about 50% by weight of the final adhesive composition.

While specific amounts of the various components can vary greatly depending on the specific intended use of the adhesive or coating composition, the substrate intended to be adhered or coated, bond strength desired, environmental conditions, etc., some general guidelines can be provided for the various broad areas of use.

For water impervious applications, either as adhesive or as coating, the total amount of bioadhesive polyphenolic protein and cross-linking agent preferably ranges from about 25 to about 90 weight percent of the composition. When the composition is to be used in an underwater environment, the bioadhesive polyphenolic protein and the cross-linking agent more preferably total from about 50 to about 80 weight percent of the composition and there is in addition, a surfactant functioning as a spreading agent in an amount of from about 20 to almost 40 weight percent.

For biomedical uses, the additives and fillers should, of course, be biomedically compatible with the organs to which the composition is to be applied. For many uses such as orthopedic repair, reconstruction or prosthetics, the total amount of bioadhesive polyphenolic protein component and cross-linking agent can range from about 30 to about 100 weight percent of the composition.

In adhesive formulations for joining electrically conductive substrates, electrically conductive additives can be present in amounts ranging from about 10 to 80 weight percent.

The following is a partial listing of specific uses for the adhesive and coating compositions of the invention. The various useful formulations are merely exemplary of the broad ranges possible for each component of the subject compositions:

(1) An adhesive which may be applied to materials under water (including fresh and salt water) or in a mixed aqueous-organic liquid environment, or to materials prior to their submergence in these environments. A useful formulation is 94% (w/w) bioadhesive polyphenolic protein, with 25 Units/mg protein mushroom tyrosinase and 6% (w/w) collagen slurry. In this and other compositions mentioned herein, the units for enzyme activity are as listed by the manufacturer and vary from lot to lot in terms of units per milligram enzyme protein. Therefore, when the cross-linking agent in a formulation is enzymatic, the designation "Unit per milligram" of adhesive formulation protein will be used, rather than weight percent. Another useful formulation includes 94% (w/w) organically synthesized peptide with the sequence [ala-lys-pro-ser-tyr-hypro-hypro-thr-thr-lys] repeated 5 times, 6% collagen and 25 Units/mg mushroom tyrosinase.

(2) A biomedical adhesive which may be used in orthopedic repair such as bone to bone repair, bone to tendon repair, bone to ligament repair, tendon to tendon repair, tendon to ligament repair, ligament to meniscus repair, meniscus repair, ligament to muscle repair, muscle repair, and any of the above to alloplastic materials for reconstruction, wound repair, or prosthetic use. An example of such formulation is 65%

(w/w) bioadhesive polyphenolic protein; 35% (w/w) collagen slurry as a filler; 6,000 Units/mg protein mushroom tyrosinase cross-linker. Another useful formulation is 58% (w/w) bioadhesive polyphenolic protein with 28,000 Units/mg protein mushroom tyrosinase cross-linker and 42% (w/w) collagen slurry as a filler.

(3) An ophthalmic adhesive which can be used to heal perforations, lacerations or incisions, whether surgically induced or trauma related, in or on the surface of the eye by acting as a tissue filler or which would facilitate the attachment of donor tissue or alloplastic materials over or in the wound. A useful formulation is 83% (w/w) bioadhesive polyphenolic protein with 18,000 Units/mg protein mushroom tyrosinase and 17% (w/w) collagen slurry. Another useful and preferred formulation is 100% (w/w) bioadhesive polyphenolic protein with 12,000 Units/mg protein mushroom tyrosinase.

(4)(a) An ophthalmic adhesive which may be used to reattach the retina to the back of the eye by direct application of the adhesive to the retina and underlying structure and/or through the direct modification of the vitreous humor or space occupied by the vitreous humor that overlies the retina, and/or by the attachment of alloplastic materials to the external surface of the eye (e.g., for scleral buckles) to modify posterior chamber dimensions and shape to influence retinal repair, or in the repair of retinal breaks resulting from traumatic or non-traumatic injury. A useful formulation for this is described in (3) above. For retinal reattachment, a spot welding application method can be used with pressure applied to the bond by increasing the volume of the vitreous humor with addition of hyaluronic acid.

(4)(b) An ophthalmic adhesive which may be used for the repair or attachment of lenses (synthetic or natural) to adjacent tissues for implantation, for repair of the lens capsule or the interior portions of the lens, and the attachment of other structures to internal or external aspects of the eye needed for repair or reconstruction, such as alloplastic materials, tissues, epi-scleral muscle, contact lenses, and the like. A useful formulation is as described in (3) above.

(4)(c) An ophthalmic adhesive which may be used for the repair, construction, reconstruction, and/or attachment of corneal component parts (epithelium, endothelium, fibroblasts, collagen stroma). A useful formulation is as described in (3) above.

(5) A dental adhesive which may be applied to hold retainers, bridges or crowns in place, to secure loose teeth, or repair broken teeth or hold filler material for caries in place to prevent further tooth decay, or as a prophylactic coating on teeth or on the site of excavated caries to prevent tooth decay. A useful formulation is 50% (w/w) bioadhesive polyphenolic protein with 18,000 Units/mg protein mushroom tyrosinase, 20% w/w collagen slurry and 30% w/w hydroxyapatite.

(6) A medical adhesive which may be used for attachment of tissue or alloplastic grafts to soft tissues for wound repair or as a prosthesis and to promote wound closure in soft tissues such as liver, spleen, stomach, esophagus, intestine, brain, skin, lung, and similar anatomic structures and their sub-components following disease and traumatic or non-traumatic injury. The adhesive can also be used in conjunction with surgical closure methods for wounds and incisions such as to assist in sealing the holes in tissues created by sutures,tacks, staples and/or the suture line being closed. This adhesive is especially useful in situations wherein fluid seepage occurs, i.e., the gastrointestinal tract, the cardiovascular system, the eye, brain, spinal column and the like. A specific formulation for intestine is 74% (w/w) bioadhesive polyphenolic protein with 18,000 Units/mg protein mushroom tyrosinase and 26% (w/w) collagen slurry as filler.

(7) A veterinary adhesive which may be used in the repair of split hooves and similar collagenous tissue, cartilagenous tissue, or some other connective tissue, bone tissue and/or soft tissue wounds in animals. A useful formulation is as described in (6) above.

(8) An anti-fouling, nonbiodegradable, nontoxicant-releasing coating that may be applied under water or prior to submersion in salt or fresh water to surfaces which are constantly exposed to a saltwater or freshwater environment to prevent the growth of microbial films, simple and complex plants, or the attachment of marine or fresh water animals on these surfaces. A specific formulation is 83% bioadhesive polyphenolic protein and 17% (w/w) mussel adjuvant protein dried and treated for 20 min. with 600 Units/mg mushroom tyrosinase. Oxidation of available L-dopa residues to a quinone mimics the phenomenon found in nature known as "quinone tanning", known to produce water-resistant, enzyme resistant, fouling resistant structures.

(9) An anti-corrosion and scale-inhibiting, water-impervious coating which may be applied under water or prior to submersion in salt or fresh water to metal and other surfaces constantly exposed to saltwater and freshwater environments to prevent corrosion, scaling, and degradation of these surfaces. A specific formulation for this application is as described in (8) with up to 10 repeated coatings of protein layers followed by oxidation.

(10) An adhesive to bond plant and tree grafts together while promoting hybrid development and genetic changes in plant materials, or for the purpose of plant repair or reconstruction following injury, and as a would closure material for repair of injury or disease. A specific formulation for this application is as described in (6).

(11) A nonbiodegradable, nontoxic adhesive coating to be applied to plant surfaces as an anti-fouling agent against blight and other fungal diseases. A specific formulation for this application is as described in (8).

(12) A conductive adhesive which may be used to join two substrates through which an electrical current will be passed without inducing excessive resistance. A specific formulation for this application comprises 65% (w/w) bioadhesive polyphenolic protein; 34% (w/w) collagen slurry; 1% (w/w) ferrocene as the conductive additive; and 6,000 Units/mg protein of mushroom tyrosinase as the crosslinker.

(13) A filter coating agent which may be used as an additive to a nitrocellulose filter or resin beads as used in column chromatography support matrices which would trap heavy metal and other contaminants from fluid, allowing the concentration of those metals or other contaminants to be determined or simply recovered with high efficiency. A useful formulation is 50% (w/w) bioadhesive polyphenolic protein with an appropriate amount of a cross-linker, and 50% (w/w) collagen. Another useful formulation is 100% (w/w) synthetic peptide with the sequence [ala-lys-pro-ser-tyr-hypro-hypro-thr-tyr-lys], repeated 5 times with 18,000 Units/mg mushroom tyrosinase.

(14) A medical adhesive which may be used to implant drugs, hormones, biological factors, medications, physiologic and/or metabolic monitoring devices, antibiotics, single cells, sheets of cells, and the like in intact tissues and/or at the sites of surgical and medical therapeutic or reparative procedures through the attachment of such agents directly to the adhesive polymer or the attachment of capsules containing such agents as prophylaxis or as follow-up to medical procedures in order to promote healing or re-establish specific metabolic functions, e.g., skin grafting, implantation of insulin-producing cells (Islets of Langerhans). A useful formulation is as described in (6).

(15) A primer which may be used for the treatment of surfaces prior to the application of a paint or adhesive which would reduce the need for meticulous cleaning of surfaces by excluding water, penetrating oxide layers and/or inhibiting metallic oxide to hydroxide conversion, penetrating organic debris layers, or penetrating microbial film layers. These surfaces would include metals, wood, plastics, silicates (cement, glass), soft and hard tissues (bones, teeth). Two useful formulations are: (a) 100% (w/w) synthetic peptide having the sequence [ala-lys-proser-tyr-hypro-hypro-thr-tyr-lys] repeated 5 times with 18,000 Units/mg mushroom tyrosinase; and (b) 50% (w/w) bioadhesive polyphenolic protein with appropriate amount of cross-linker and 50% (w/w) collagen.

(16) An additive which may be used with any other adhesive formulation that would impart the specific properties herein described to another adhesive. These properties include: compatibility with hygroscopic, saline substrates in and around living tissues, marine environments, and the moist environments of fresh waters: the ability to impart anti-corrosion, anti-scaling, and/or anti-fouling properties to the resulting composition; the ability to complex with metals, ions, other polyamines, and biological substances. A useful formulation is 100% (w/w) synthetic peptide having the sequence [ala-lys-pro-ser-tyr-hypro-hypro-thr-tyr-lys] repeated 5 times, with 18,000 Units/mg mushroom tyrosinase, incubated together for 5 minutes at room temperature before addition to another adhesive system at a ratio of 1:10 (w/w).

The following specific examples are here given to illustrate further the various uses of the adhesive compositions of this invention. They are included here for illustrative purposes only and are not to be construed as limitations on the invention herein claimed. As one skilled in the art understands, many variations and modifications may be made to the invention herein described which fall within the spirit and scope of this invention.

Examples 1 - 3

To demonstrate the adhesive function of bioadhesive formulations, two 1-cm wide strips of aluminum foil were bonded together in a lap shear test using the following formulation: 74% bioadhesive polyphenolic protein (4.3 mg/ml in water); 26% (w/w) collagen slurry as a filler (25% w/w collagen in pH 6.5, 0.1 M phosphate buffer); 18,600 Units/mg mushroom tyrosinase cross-linker (216 Units/μl in pH 6.5, 0.1 M phosphate buffer). The above formulation was applied evenly with a syringe over a 1 square centimeter section at the end of each aluminum foil strip, and the two coated sections then joined so that the bonded area of overlap was 1 $cm^2$. The bond was allowed to set 2.5 hours at room temperture (21°C), and was then tested and found to provide a shear strength of 320 $gm/cm^2$.

In a second example, two 1-cm strips of aluminum foil were bonded together with the bioadhesive formulation described above so that the area of overlap was 1 square centimeter. The sample was allowed to cure 24 hours at room temperature (21°C) and the measured shear strength was in excess of 916 $gm/cm^2$.

In a third example, aluminum foil samples prepared as above using the bioadhesive formulation were kept under water for a 1-hour setting period. The bioadhesive polyphenolic protein formulation was found to provide a shear strength at least fivefold greater than that achieved with a cyanoacrylate control.

Examples 4 - 7

To establish the biomedical applications of the bioadhesive formulation, a formulation was prepared as follows: 65% (w/w) bioadhesive polyphenolic protein ( 5.5 mg/ml in water); 35% (w/w) collagen slurry as a filler (25% w/w in pH 7, 0.1 M phosphate buffer); 6000 Units/mg mushroom tyrosinase cross-linker (216 Units/µl in pH 7, 0.1 M phosphate buffer). A bovine meniscus which had been surgically severed longitudinally was subsequently bonded together with the above formulation applied evenly with a syringe over the two cut surfaces of about 4 square centimeters each. The bond was allowed to set 1 hour at 37°C, and was then tested and found to provide a tensile strength of 85 gm total, or 21.2 gm/$cm^2$.

Canine meniscus was bonded with a second bioadhesive formulation. The formulation contained 65% (w/w) bioadhesive polyphenolic protein (5.5 mg/ml in water); 35 % (w/w) collagen slurry (25% w/w in pH 7, 0.1 M phosphate buffer); 12,600 Units/mg mushroom tyrosinase cross-linker (216 Units/µl in pH 7, 0.1 M phosphate buffer). The meniscus, which had been surgically severed longitudinally, was subsequently bonded together with the formulation applied evenly with a syringe over the two (1.5 $cm^2$ each) cut surfaces. The bond was allowed to set 30 minutes at 37°C, and was then tested and found to provide a tensile strength of 13 gm total, or 8.7 gm/$cm^2$.

In yet another example, spinal vertebrae were used as a model for bone-to-bone repair. The bioadhesive formulation employed contained 59% (w/w) bioadhesive polyphenolic protein (4.3 mg/ml in water); 41% collagen slurry (25% w/w in pH 7, 0.1 M phosphate buffer); 14,800 Units/mg mushroom tyrosinase cross-linker (216 Units/µl in pH 7, 0.1 M phosphate buffer). Two spinal vertebrae were bonded together with the formulation applied evenly with a syringe over the two (0.5 $cm^2$ each) opposing surfaces. The bond was allowed to set 43 minutes at 37°C, and was then tested and found to provide a tensile strength of 38 gm total, or 76 gm/$cm^2$.

The preceding example with spinal vertebrae was repeated with an adhesive formulation containing different ratios of the same components, namely, 74% (w/w) bioadhesive poyphenolic protein (4.3 mg/ml in water); 26% (w/w) collagen slurry (25% w/w in pH 7,. 0.1 M phosphate buffer); 18,400 Units/mg mushrom tyrosinase cross-linker (216 Units/µl in pH 7, 0.1 M phosphate buffer). Two spinal vertebrae were bonded together with the formulation applied evenly with a syringe over the two (0.5 $cm^2$ each) opposing surfaces. The bond was allowed to set 47 minutes at 37°C, and was then tested and found to provide a tensile strength of 44 gm total, or 88 gm/$cm^2$.

Example 8

Examples 8 and 9 demonstrate the advantages of pre-treating tissues with the bioadhesive polyphenolic protein prior to bonding.

Two 1-cm wide strips of calf stomach with a tissue thickness of approximately 1.5 mm were bonded together end-t-end using the following procedure. First, the tissue was primed by applying 50 l of solution to the two surfaces (50% (w/w) bioadhesive polyphenolic protein, 50% (w/w ) collagen). The surfaces were allowed to set briefly and cyanoacrylate adhesive was applied and allowed to set for 51 minutes at 37°C. The measured tensile strength of the bond was found to be 1,230 gm/$cm^2$.

Example 9 for Comparison

In a control experiment relating to the above Example 8, two 1-cm-wide strips of calf stomach with a tissue thickness of 1.5 mm were bonded together end-to-end using cyanoacrylate. The bond was allowed to set for 51 min. at 37°C, and was then tested and found to provide a tensile strength of 85 gm, or 570 gm/$cm^2$.

Example 10

Antifouling applications were illustrated using a cellulose paper substrate coated with different preparations of bioadhesive polyphenolic protein with and without oxidation with mushroom tyrosinase. The oxidation of L-dopa to the quinone produces what in nature is referred to as quinone-tanned structures. Two examples of quinone tanning which achieves environmental stability are the tanning of hides in leather processing and the brown, rigid skate egg cases. Cellulose strips (7.5 cm x 2.5 cm) were treated in the following manner: (1) two strips were soaked and dried 9 times in a solution containing bioadhesive polyphenolic protein at 2.9 mg/ml in 5% v/v acetic acid; (2) two strips soaked and dried 9 times in bioadhesive polyphenolic protein preparation in (1), with each drying followed by a 10-minute incubation in mushroom tyrosinase (43 Units/µl in phosphate buffer, 0.1 M, pH 7.0) (3) two strips soaked and dried 9 times in bioadhesive polyphenolic protein preparation at 2.9 mg/ml mixed 50:50 with an adjuvant L-dopa-containing protein at 2.14 mg/ml in sodium acetate (0.1 M, pH

5.0) followed by enzyme oxidation as in (2); (4) two strips as untreated cellulose controls. These strips were then suspended in a saltwater aquarium and observed for one week. Both the cellulose controls and the unoxidized bioadhesive polyphenolic protein showed marked signs of decomposition and microbial growth in four days, while both oxidized preparations were intact after seven days.

EXAMPLE 11

Isolation and Purification via Extraction of Bioadhesive Polyphenolic protein.

300 grams of marine mussel, M. Edulis, feet are combined with 900 mls of neutral salt buffer which contains 1M sodium chloride, 0.05M tris (hydroxymethyl) aminomethane (pH 7.5), 1 mM phenylmethylsulfonylfluoride, 10mM N-ethylmaleimide, 0.025 M ethylenediamine tetraacetic acid and 1 mM potassium cyanide plus 9 mls of antifoam concentrate in a commercial blender on high speed and thoroughly blended, precipitating the bioadhesive polyphenolic protein. The mixture is centrifuged at 10 K rpm for 15 minutes. The pellet is resuspended in 900 mls of 5% acetic acid using the blender on high speed. Bioadhesive polyphenolic protein remains in the supernatant during centrifugation at 10 K rpm for 45 minutes. The approximately 1000 mls of supernatant is put into an ice both with continual stirring. 5 mls of 2M sodium borate plus 95 mls of 5 M sodium chloride are added to the stirring supernatant. This mixture is centrifuged at 10K rpm for 15 minutes. The new supernatant is treated identically as above with the addition of four times as much 2M sodium borate and 5 M sodium chloride. Once again, the mixture is centrifuged at 10K for 15 minutes. The pellet is resuspended in the following mixture: 7.5 mls of 2M sodium borate, 50 mls of 5M sodium chloride, 50 mls of distilled water, 37.5 mls of 8M urea in 5% acetic acid, and 5.6 mls of concentrated acetic acid. The mixture is slowly stirred for approximately 16 hours. The suspension is centrifuged at 10 K rpm for 15 minutes. The supernatant is saved and dialyzed (18-12K molecular weight cut-off membranes) against 5% acetic acid for approximately 16 hours. Amino acid analysis establishes that the extract contains 45% pure bioadhesive polyphenolic protein. The purity of the extract is governed by the number of extractions effected. The yield of pure bioadhesive polyphenolic protein decreases as the number of extiactions increases. All procedures described herein were conducted at 4°C.

Further chromatographic purification:

Using liquid chromatography, SE Sephadex resins retain polyphenolic proteins in 5.5% Guanidine hydrochloride (GuHCl) in 5% acetic acid. The protein is then eluted from the resin with a gradient of 5.5 - 20% GuHCl in acetic acid, the peak areas pooled and dialyzed against 5% acetic acid to remove the GuHCl. Storage of the proteins is most stable at 4°C in 5% acetic acid. Prior to its use as an adhesive, in vivo or in contact with live cells, bioadhesive polyphenolic proteins must be dialyzed against water to raise the pH of the solution to near neutrality and the preparation must be concentrated to between 3 and 10 mg/ml. This is accomplished using an ultrafiltration membrane with pore size exclusion limits of 30,000 or less. This is not necessary when bioadhesive polyphenolic proteins are dried onto an inert substrate prior to use.

EXAMPLE 12

This example demonstrates that pure bioadhesive polyphenolic protein alone does not impart maximal adhesive strength for bonding without the presence of additional crosslinking agents and fillers.

(I). A constant amount of protein, either 95% pure bioadhesive polyphenolic protein alone, boiled casein alone, or a combination of bioadhesive polyphenolic protein and casein were applied to strips of aluminum foil and tested for bond strength. Total protein per bond area was kept at 20 ugrams of protein delivered in 4 µl of 5% acetic acid and was applied to a bond area of 1.3 $cm^2$. The formulation of proteins applied to the bonds were: 20 µgrams of bioadhesive polyphenolic protein alone, 20 µgrams of boiled casein alone, and a mixture of 10 µgrams of bioadhesive polyphenolic protein plus 10 µgrams of boiled casein. Bonds were allowed to cure for one hour, then measured by clamping the strips between a pressure guage (0-500 or 0-5000 Gm range) and a geared motor with a piston producing strain at a rate of 25 grams per second. All procedures were performed at room temperature. The data are the average of five assays per formulation. For casein alone, 275; for pure bioadhesive polyphenolic protein alone, 214; and for the combination of the two, 1026 gm/1.3 $cm^2$. The data indicates that enhanced results can be obtained when a filler is admixed with the bioadhesive polyphenolic protein , as indicated by the increase in strength of the mixtures' bond by a factor of 2 greater than the sum of the two proteins used alone.

(II.) In order to demonstrate the effect of a crosslinking agent on the bioadhesive polyphenolic protein formulation, a cross-linking agent, 3,3'-dithiobis(sulfosuccinimidylproprionate) (DTSSP), was incorporated

into the formulation at varying concentrations and compared to a control having no cross-linking agent. DTSSP is a water soluble cross-linker of lysines with optimum activity at pH = 7. Both bioadhesive polyphenolic protein and casein were prepared at 10 mg/ml in distilled water(pH = 6) and in 0.1 M phosphate buffer (pH = 7). DTSSP was prepared in 0.1 M phosphate buffer (pH = 7) at a concentration of 400 μM. Mixtures were placed on foil and measured as described above, with each number representing the average of five trials.

0.1 M Phosphate Buffer pH 7

| μg bioadhesive polyphenolic protein | μg casein | DTSSP (mM final concentration | (wt. %) | shear strength ($gm/cm^2 x1.3$) |
|---|---|---|---|---|
| 10 | 10 | 0 | (0.0%) | 250 |
| 10 | 10 | 100 | (1.4%) | 1310 |
| 10 | 10 | 50 | (0.7%) | 1560 |
| Distilled $H_2O$ | | | | |
| 10 | 10 | 0 | (0.0%) | 775 |
| 10 | 10 | 50 | (0.7%) | 1690 |

The data show that an increase in pH to accommodate the cross-linker DTSPP decreases the strength of bioadhesive polyphenolic protein plus casein formulation to 250 and 775 (compared to 1026 gm/1.3 $cm^2$ in acid pH). In both cases, it can readily be seen that DTSSP significantly enhances bond strength.

EXAMPLE 13

In this example, bovine corneas were used to demonstrate that while pure bioadhesive polyphenolic protein does have some adhesivity, this adhesivity can be greatly enhanced by the addition of, in this example, a crosslinking agent. Bovine corneas were removed from unnucleated eyes and scraped with a scalpel for removal of endothelial and epithelial cells. Tissue strips (2 x 1 $cm^2$) were prepared and anterior to posterior bonds (1 $cm^2$ in area) were tested. Pure bioadhesive polyphenolic protein (3.2 μ g/μ l in water) was applied with a microliter pipet to both bond sides, 8.1 μ l per side, and spread over the 1 $cm^2$ area. The two tissues were immediately joined, and incubated for 20 minutes at room temperature with gentle (5 gm) pressure under conditions which keep the tissue moist. The tissues were clamped, suspended vertically and small weights added at a rate of about 3 gm per second. Bioadhesive polyphenolic protein alone with a total of 51.8 μ g/$cm^2$ tissue yielded a strength of 7 gm/$cm^2$. The same amount of protein, 51.8 μ g with added catechol oxidase crosslinking agent at 11.76 U/ μ g bond protein yielded a strength of 68 gm/$cm^2$.

Employing the chromatographic purification procedure of the bioadhesive polyphenolic protein set forth in example 11 and repeating the test set forth hereinabove therewith, bond strength of 150-200 gm/$cm^2$ were obtained.

EXAMPLE 14

This example demonstrates the use of bioadhesive polyphenolic protein for sealing ophthalmic perforations using alloplastic materials. Bovine corneas and HYPAN discs (HYPAN is a trademark of Kingston Technologies, Dayton, New Jersey) were employed. HYPAN discs are useful for this application because they

are manufactured from gas permeable polyacrylonitrile block copolymer hydrogels containing 90% water.

Epithelial cells are scraped off over a region about 15-20 mm in diameter on the bovine cornea using a scalpel. A perforation is prepared by jabbing in the center of the scraped corneal area with a scalpel. An 18 gauge needle is inserted into the anterior chamber by puncturing the cornea near the corneal/scleral junction and checked for fluid path continuity by pressurizing the anterior chamber using manual pressure and looking for fluid leakage through the needle. The scraped area is then rinsed with deionized water. Excess water is then blown off.

10 µl of chromatographically purified bioadhesive polyphenolic protein (5.8 mg/ml in $H_2O$) and 0.94 µl of catechol oxidase (648 µ/µl in 0.1M phosphate buffer) are mixed and applied in the immediate area of the perforation. A HYPAN disk is applied and smoothed out over the cornea insuring no folds in the cornea are present under the disc. A dialysis bag is applied over the joint and the joint is allowed to cure for the time periods ranging from 5 to 20 minutes. A manometer is attached to the needle and the dialysis bag is removed.

The eye is pressurized to about 120"/min, while monitoring leakage and pressure. The pressure recorded is the reading attained at the first sign of leakage. The pressure sustained equals (65"-1"-reading). The pressure is converted to mm by dividing by 0.535. The data is the average of seven assays wherein the cure time ranges from five to twenty minutes. The average mm of mercury sustained was greater than 93. The results show that bioadhesive polyphenolic protein is an excellent adhesive for sealing ophthalmic incisions and perforations using an alloplastic material.

## Claims

### Claims for the following Contracting States: BE, CH, DE, FR, GB, IT, LI, LU, NL, SE

1. An adhesive or coating formulation comprising:

(1) a bioadhesive polyphenolic protein component having from about 5 to about 99 weight percent of a proteinaceous substance comprising from 1 to about 1,000 of the following repeating decapeptide unit:

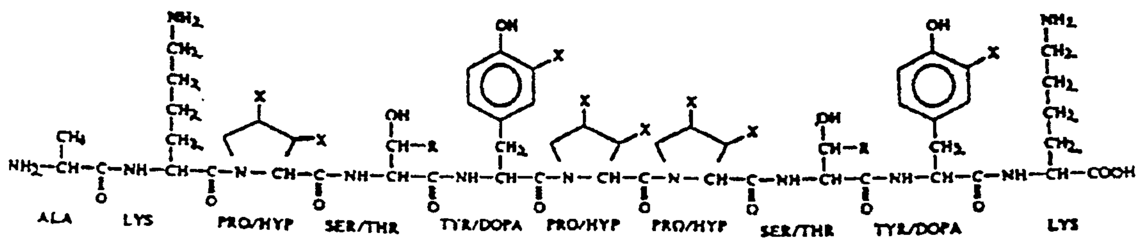

in which each X is hydrogen or hydroxyl, and each R is hydrogen or methyl;

(2) from about 0.1 to about 40 weight percent of a cross-linking agent which promotes cross-linking of the decapeptide;

(3) one or more additives which promote the desired properties of the formulation, said additives comprising at least one surfactant and being present in an amount of from 0% to about 90% by weight, and

(4) a filler compatible with the intended use of the formulation, said filler being present in an amount of from 0% to about 50% by weight.

2. A water-impervious adhesive or coating formulation according to Claim 1 in which the bioadhesive polyphenolic protein component and the cross-linking agent together are present in an amount ranging from about 25 to about 90 weight percent of the formulation.

3. A water-impervious coating formulation according to Claim 2 for use in an underwater environment in which:

(a) the bioadhesive polyphenolic protein component and the cross-linking agent are together present in an amount ranging from about 50 to about 80 weight percent; and

(b) the additives include a surfactant functioning as a spreading agent in an amount ranging from about 20 to about 40 weight percent of the formulation.

4. A formulation according to Claim 3 in which the surfactant is sodium dodecylsulfate or sodium dodecylbenzenesulfonate.

5. An adhesive formulation according to Claim 1 for biomedical use in which each additive and filler is biomedically compatible with the organs to which the formulation is applied.

6. A biomedical adhesive formulation according to Claim 5 for use in orthopedic repair, reconstruction or prosthetics, in which;

(a) the bioadhesive polyphenolic component and the crosslinking agent are together present in an amount ranging from about 30 to about 70 weight percent;

(b) the filler is present in an amount ranging from about 30 to about 60 weight percent.

7. A biomedical adhesive formulation according to Claim 6 in which the filler is selected from the group consisting of collagen, hyaluronic acid, chondroitin sulfate, elastin, laminin, casein, hydroxyapatite, or a similar naturally occurring proteinaceous, inorganic, or mucopolysaccharide substance.

8. A formulation according to Claim 7 in which the filler is collagen.

9. A formulation according to Claim 7 in which the crosslinking agent is mushroom tyrosinase.

10. An adhesive formulation according to Claim 1 for use in ophthalmic surgery in which the bioadhesive polyphenolic protein component and the cross-linking agent are together present in an amount varying from about 70 to about 100 weight percent.

11. An adhesive formulation according to Claim 10 in which the cross-linking agent is mushroom tyrosinase.

12. An adhesive formulation according to Claim 10 for sealing ophthalmic incisions and perforations with alloplastic materials in which the bioadhesive polyphenolic protein component and the crosslinking agent are together present in an amount varying from about 3 to about 100 weight percent and a collagen filler is incorporated in amounts ranging from 0 to about 40 weight percent.

13. An adhesive and coating formulation according to Claim 1 for bonding plant materials or coating plant surfaces in which each additive and filler is biochemically compatible with the plant to which the formulation is applied.

14. An adhesive formulation according to Claim 1 for joining substrates through which an electric current is to be passed, said formulation additionally containing electrically conductive additives in an amount ranging from about 10 to about 80 weight percent.

15. A method for adhering two or more substrates in an underwater environment which comprises applying to the adherent surfaces either under water or prior to submersion in water an adhesive formulation according to Claim 1.

16. A method for coating an underwater surface so as to prevent the growth of microbes or plants thereon, or the attachment thereto of marine animals, said method comprising applying to said surface a formulation according to Claim 3.

17. A method for preventing corrosion of an underwater surface which comprises applying to said surface a coating formulation according to Claim 3.

18. A method of priming a surface for subsequent application of a coating or adhesive which method comprises applying to said surface a formulation according to Claim 1.

19. Formulation according to Claim 6 for application to a surface on which adhesion is sought for use in orthopedic repair and reconstruction, for adhering bone, tendon, ligament, meniscus, muscle, each to each other or adhering any of them to alloplastic materials.

20. Formulation according to Claim 19 in which the biocompatible filler is collagen.

21. Formulation according to Claim 19 in which the crosslinking agent is mushroom tyrosinase.

22. Formulation according to Claim 5 for application to a surface on which adhesion or repair is sought for use in dentistry for adhering retainers, bridges, crows or filler material to tooth structure, for securing loose teeth and for repairing broken teeth.

23. Formulation according to Claim 5 for application to a surface onto which thereafter donor tissue or alloplastic grafts is to be attached for promoting wound and incision closure and for preventing bacterial contamination in soft tissue.

24. Formulation according to Claim 5 for application to a site to be sealed for sealing wound or incision closures and the regions adjacent thereto which have been punctured by associated surgical closure devices to substantially reduce fluid seepage therethrough.

25. Formulation according to Claim 5 for application to a surface to be treated for treating wounds, fractures and dislocations in animals.

26. Formulation according to Claim 5 for use as an adhesive for adhering a prosthesis or medical device containing drugs, medications, single cells or sheets of cells, or electronic circuitry, to be implanted in a patient to the desired site in the patient.

27. Formulation according to Claim 10 for use as an adhesive to be applied to surfaces onto which thereafter alloplastic or donor tissue is to be attached for repair or attachment of alloplastic or donor tissue in ophthalmic surgery.

28. Formulation according to Claim 10 for application to ophthalmic incisions or punctures to be sealed with alloplastic material.

29. A method for treating plants for the purpose of repairing injury, reconstruction, wound closure, grafting or promoting hybridization or genetic alteration, which comprises applying to surfaces which are to be adhered a formulation according to Claim 13.

30. A method for protecting plants against attack from fungal diseases which comprises applying to the surface areas of the plant subject to fungal attack a coating formulation according to Claim 13.

31. A method for joining two substrates through which an electric current is to be passed which comprises adhering the substrates to each other with an adhesive formulation according to Claim 14.

32. A method for separating heavy metals and contaminants from fluids which comprises applying to a nitrocellulose filter or to resin beads used as support matrices in ultrafiltration procedures and column chromatography, respectively, a coating formulation according to Claim 1.

**Claims for the following Contracting States: AT, ES, GR**

1. Process for manufacturing an adhesive or coating by producing a formulation comprising:

(1) a bioadhesive polyphenolic protein component having from about 5 to about 99 weight percent of a proteinaceous substance comprising from 1 to about 1,000 of the following repeating decapeptide unit:

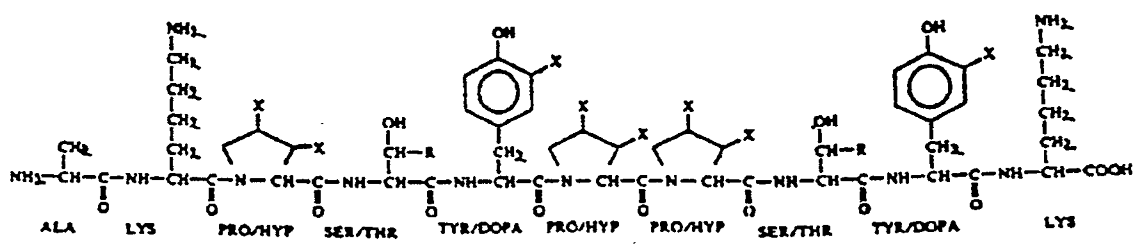

in which each X is hydrogen or hydroxyl, and each R is hydrogen or methyl;

(2) from about 0.1 to about 40 weight percent of a cross-linking agent which promotes cross-linking of the decapeptide;

(3) one or more additives which promote the desired properties of the formulation, said additives comprising at least one surfactant and being present in an amount of from 0 % to about 90 % by weight, and

(4) a filler compatible with the intended use of the formulation, said filler being present in an amount of from 0 % to about 50 % by weight.

2. Process according to Claim 1 in which for providing a water-impervious adhesive or coating, the bioadhesive polyphenolic protein component and the cross-linking agent together are used in an amount ranging from about 25 to about 90 weight percent of the formulation.

3. A process according to Claim 2 for providing a formulation for use in an underwater environment in which:

(a) the bioadhesive polyphenolic protein component and the cross-linking agent together are used in an amount ranging from about 50 to about 80 weight percent; and

(b) a surfactant functioning as a spreading agent is used in the additives in an amount ranging from about 20 to about 40 weight percent of the formulation.

4. A process according to Claim 3 in which sodium dodecylsulfate or sodium dodecylbenzenesulfonate is used as the surfactant.

5. A process according to Claim 1 for manufacturing a biomedically usable adhesive or coating in which exclusively such additives and fillers are used which are biomedically compatible with the organs to which the formulation is to be applied.

6. A Process according to Claim 5 for manufacturing an adhesive usable in orthopedic repair, reconstruction or prosthetics, in which:

(a) the bioadhesive polyphenolic component and the cross-linking agent together are used in an amount ranging from about 30 to about 70 weight percent;

(b) the filler is used in an amount ranging from about 30 to about 60 weight percent.

7. A process according to Claim 6 in which a filler is used which is selected from the group consisting of collagen, hyaluronic acid, chondroitin sulfate, elastin, laminin, casein, hydroxyapatite, or a similar naturally occurring proteinaceous, inorganic, or mucopolysaccharide substance.

8. A process according to Claim 7 in which collagen is used as the filler.

9. A process according to Claim 7 in which mushroom tyrosinase is used as the cross-linking agent.

10. A process according to Claim 1 for manufacturing an adhesive for use in ophthalmic surgery in which the bioadhesive polyphenolic protein component and the crosslinking agent together are used in an amount varying from about 70 to about 100 weight percent.

11. A process according to Claim 10 in which mushroom tyrosinase is used as the cross-linking agent.

12. A process according to Claim 10 for manufacturing an adhesive usable for sealing ophthalmic incisions and perforations with alloplastic materials in which the bio-adhesive polyphenolic protein component and the crosslinking agent together are used in an amount varying from about 3 to about 100 weight percent and a collages filler is incorporated in amounts ranging from 0 to about 40 weight percent.

13. Use of an adhesive and coating formulation produced according to the process as defined in Claim 1 for bonding plant materials or coating plant surfaces in which each additive and filler is biochemically compatible with the plant to which the formulation is applied.

14. Use of an adhesive formulation produced according to the process as defined in Claim 1 for joining substrates through which an electric current is to be passed, said formulation additionally containing electrically conductive additives in an amount ranging from about 10 to about 80 weight percent.

15. A method for adhering two or more substrates in an underwater environment which comprises applying to the adherent surfaces either under water or prior to submersion in water an adhesive formulation produced according to Claim 1.

16. A method for coating an underwater surface so as to prevent the growth of microbes or plants thereon, or the attachment thereto of marine animals, said method comprising applying to said surface a formulation produced according to Claim 3.

17. A method for preventing corrosion of an underwater surface which comprises applying to said surface a coating formulation produced according to Claim 3.

18. A method of priming a surface for subsequent application of a coating or adhesive which method comprises applying to said surface a formulation produced according to Claim 1.

19. In orthopedic repair and reconstruction, a method for adhering bone, tendon, ligament, meniscus, muscle, each to each other or adhering any of them to alloplastic materials, which method comprises applying to a surface on which adhesion is sought a formulation according to Claim 6.

20. A method according to Claim 19 in which, in the formulation, the biocompatible filler is collagen.

21. A method according to Claim 19 in which, in the formulation, the cross-linking agent is mushroom tyrosinase.

22. A process according to Claim 1 for manufacturing an adhesive for adhering retainers, bridges, crowns or filler material to tooth structure, for securing loose teeth and for repairing broken teeth, in which exclusively such additives and fillers are used which are biomedically compatible with surfaces with which the adhesive comes into contact.

23. A process according to Claim 1 for manufacturing an adhesive for attaching donor tissue or alloplastic crafts to promote wound and incision closure and prevent bacterial contamination in soft tissue in which exclusively such additives and fillers are used which are biomedically compatible with surfaces with which the adhesive comes into contact.

24. A process according to Claim 1 for manufacturing a sealing formulation for sealing wound or incision closures and the regions adjacent thereto which have been punctured by associated surgical closure devices to substantially reduce fluid seepage therethrough in which exclusively such additives and fillers are used which are biomedically compatible with surfaces with which the adhesive comes into contact.

25. A process according to Claim 1 for manufacturing a formulation for treating wounds, fractures and dislocations in animals in which exclusively such additives and fillers are used which are biomedically compatible with surfaces with which the adhesive comes into contact.

26. A process according to Claim 1 for manufacturing an adhesive for implanting in a patient a prosthesis or medical device containing drugs, medications, single cells or sheets of cells, or electronic circuitry, in which exclusively such additives and fillers are used which are biomedically compatible with surfaces with which the adhesive comes into contact.

27. In ophthalmic surgery, a method for repair or attachment of alloplastic or donor tissue which comprises applying to the surfaces to be adhered an adhesive formulation according to Claim 10 and attaching said alloplastic or donor tissue thereon.

28. A method for sealing ophthalmic incisions or punctures with alloplastic material which comprises applying to the surface to be treated a formulation according to Claim 10 and attaching said alloplastic material thereto.

29. A method for treating plants for the purpose of repairing injury, reconstruction, wound closure, grafting or promoting hybridization or genetic alteration, which comprises applying to surfaces which are to be adhered a formulation comprising:

(1) a bioadhesive polyphenolic protein component having from about 5 to about 99 weight percent of a proteinaceous substance comprising from 1 to about 1,000 of the following repeating decapeptide unit;

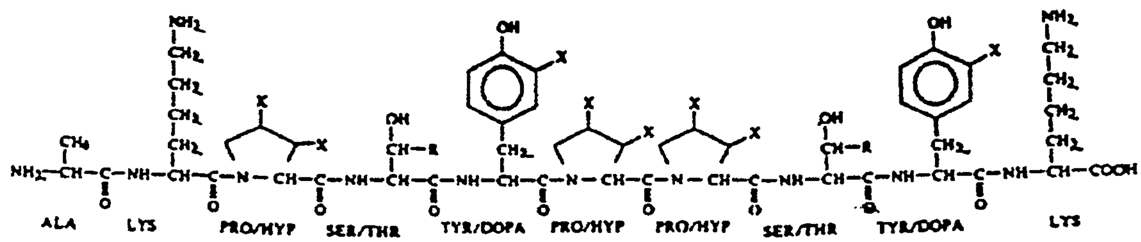

in which each X is hydrogen or hydroxyl, and each R is hydrogen or methyl;
(2) from about 0.1 to about 40 weight percent of a cross-linking agent which promotes cross-linking of the decapeptide;
(3) one or more additives which promote the desired properties of the formulation, said additives comprising at least one surfactant and being present in an amount of from 0 % to about 90 % by weight, and
(4) a filler compatible with the intended use of the formulation, said filler being present in an amount of from 0 % to about 50 % by weight, and in which each additive and filler is biochemically compatible with the plant to which the formulation is applied.

30. A method for protecting plants against attack from fungal diseases which comprises applying to the surface areas of the plant subject to fungal attack a coating formulation comprising:
(1) a bioadhesive polyphenolic protein component having from about 5 to about 99 weight percent of a proteinaceous substance comprising from 1 to about 1,000 of the following repeating decapeptide unit:

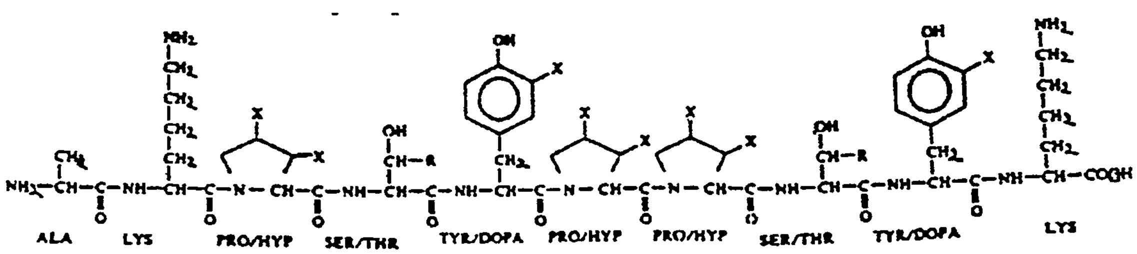

in which each X is hydrogen or hydroxyl, and each R is hydrogen or methyl;
(2) from about 0.1 to about 40 weight percent of a cross-linking agent which promotes cross-linking of the decapeptide;
(3) one or more additives which promote the desired properties of the formulation, said additives comprising at least on surfactant and being present in an amount of from 0 % to about 90 % by weight, and
(4) a filler compatible with the intended use of the formulation, said filler being present in an amount of from 0 % to about 50 % by weight, and in which each additive and filler is biochemically compatible with the plant to which the formulation is applied.

31. A method for joining two substrates through which an electric current is to be passed which comprises adhering the substrates to each other with an adhesive formulation comprising:
(1) a bioadhesive polyphenolic protein component having from about 5 to about 99 weight percent of a proteinaceous substance comprising from 1 to about 1,000 of the following repeating decapeptide unit:

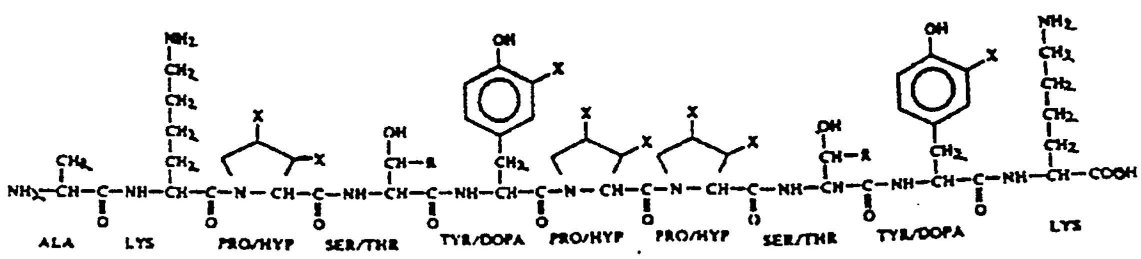

in which each X is hydrogen or hydroxyl, and each R is hydrogen or methyl;

(2) from about 0.1 to about 40 weight percent of a cross-linking agent which promotes cross-linking of the decapeptide;

(3) one or more additives which promote the desired properties of the formulation, said additives comprising at least one surfactant and being present in an amount of from 0 % to about 90 % by weight, and

(4) a filler compatible with the intended use of the formulation, said filler being present in an amount of from 0 % to about 50 % by weight, said formulation additionally containing electrically conductive additive in an amount ranging from about 10 to about 80 weight percent.

32. A method for separating heavy metals and contaminants from fluids which comprises applying to a nitrocellulose filter or to resin beads used as support matrices in ultrafiltration procedures and column chromatography, respectively, a coating formulation comprising:

(1) a bioadhesive polyphenolic protein component having from about 5 to about 99 weight percent of a proteinaceous substance comprising from 1 to about 1,000 of the following repeating decapeptide unit:

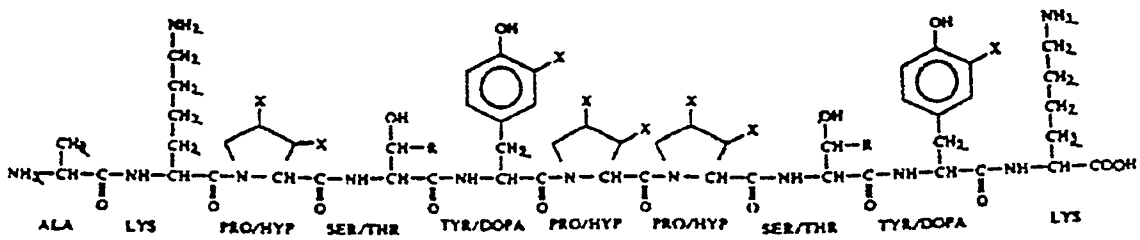

in which each X is hydrogen or hydroxyl, and each R is hydrogen or methyl;

(2) from about 0.1 to about 40 weight percent of a cross-linking agent which promotes cross-linking of the decapeptide;

(3) one or more additives which promote the desired properties of the formulation, said additives comprising at least one surfactant and being present in an amount of from 0 % to about 90 % by weight, and

(4) a filler compatible with the intended use of the formulation, said filler being present in an amount of from 0 % to about 50 % by weight.

**Patentansprüche**

**Patentansprüche für folgende Vertragsstaaten: BE, CH, DE, FR, GB, IT, LI, LU, NL, SE**

1. Klebemittel- oder Beschichtungsmasse mit

(1) einer bioadhäsiven, polyphenolischen Proteinkomponente, welche einen Anteil von ca. 5 bis ca. 99 Gew. % einer Proteinsubstanz mit 1 bis ca. 1000 Einheiten der folgenden Decapeptid-Struktureinheit umfaßt:

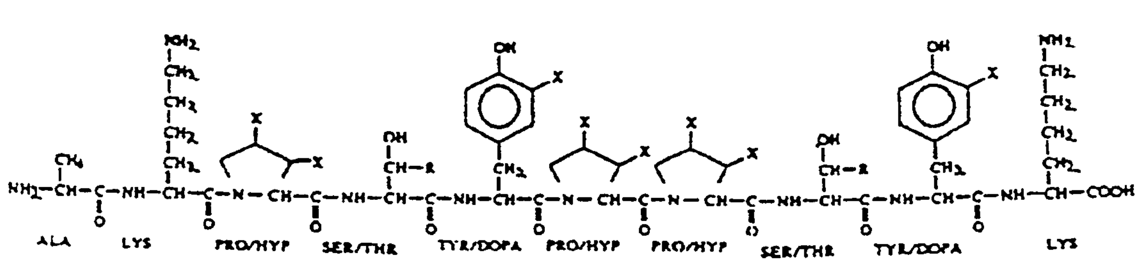

in welcher jedes X Wasserstoff oder eine Hydroxyl-Gruppe und jedes R Wasserstoff oder eine Methyl-Gruppe ist;

(2) mit ca. 0,1 bis ca. 40 Gew. % eines Vernetzungsmittels, welches die Vernetzung des Decapeptids fördert;

(3) mit einem oder mehreren Additiven, die die gewünschten Eigenschaften der Masse unterstützen, wobei die Additive mindestens eine oberflächenaktive Substanz umfassen und mit einem Anteil von 0 bis ca. 90 Gew. % enthalten sind, und

(4) mit einem für den vorgesehenen Verwendungszweck der Masse verträglichen Füllstoff, wobei der Füllstoff mit einem Anteil von 0 bis ca. 50 Gew. % enthalten ist.

2. Wasserundurchlässige Klebemittel- oder Beschichtungsmasse nach Anspruch 1, bei welcher die bioadhäsive polyphenolische Proteinkomponente und das Vernetzungsmittel zusammen mit einem Anteil im Bereich von ca. 25 bis 90 Gew. % der Masse enthalten sind.

3. Wasserundurchlässige Beschichtungsmasse nach Anspruch 2 zur Verwendung in einer Unterwasser-Umgebung, wobei

(a) die bioadhäsive polyphenolische Proteinkomponente und das Vernetzungsmittel zusammen mit einem Anteil im Bereich von ca. 50 bis ca. 80 Gew. % enthalten sind; und

(b) die Additive eine oberflächenaktive Substanz umfassen, welche als Netzmittel wirkt und welche mit einem Anteil im Bereich von ca. 20 bis ca. 40 Gew. % der Masse enthalten ist.

4. Masse gemäß Anspruch 3, bei welcher die oberflächenaktive Substanz Natriumdodecylsulfat oder Natriumdodecylbenzolsulfonat ist.

5. Klebemittelmasse nach Anspruch 1 für biomedizinische Zwecke, in welcher sowohl das Additiv als auch der Füllstoff biomedizinisch mit den Organen, auf die die Masse angewendet wird, verträglich sind.

6. Biomedizinische Klebemittelmasse nach Anspruch 5 zur Verwendung bei der orthopädischen Instandsetzung, Rekonstruktion oder Prothetik, bei der

(a) die bioadhäsive polyphenolische Komponente und das Vernetzungsmittel zusammen mit einem Anteil im Bereich von ca. 30 bis ca. 70 Gew. % enthalten sind;

(b) der Füllstoff mit einem Anteil im Bereich von ca. 30 bis ca. 60 Gew. % enthalten ist.

7. Biomedizinische Klebemittelmasse nach Anspruch 6, bei welcher der Füllstoff aus der Gruppe von Collagen, Hyaluronsäure, Chondroitinsulfat, Elastin, Laminin, Casein, Hydroxyapatit oder einer ähnlichen, natürlich vorkommenden proteinhaltigen, anorganischen oder Mucopolysaccharid-Verbindung stammt.

8. Masse nach Anspruch 7, bei welcher der Füllstoff Collagen ist.

9. Masse nach Anspruch 7, bei welcher das Vernetzungsmittel Pilz-Tyrosinase ist.

10. Klebemittelmasse nach Anspruch 1 zur Verwendung in der Augenchirurgie, bei welcher die bioadhäsive, polyphenolische Protein-Komponente und das Vernetzungsmittel zusammen mit einem Anteil im Bereich von ca. 70 bis ca. 100 Gew. % enthalten sind.

11. Klebemittelmasse nach Anspruch 10, bei welcher das Vernetzungsmittel Pilz-Tyrosinase ist.

12. Klebemittelmasse nach Anspruch 10 zum Abdichten von Augeninzisionen und -perforationen mit alloplastischen Materialien, wobei die bioadhäsive polyphenolische Protein-Komponente und das Vernetzungsmittel zusammen mit einem Anteil im Bereich von ca. 3 bis ca. 100 Gew. % vorhanden sind und ein Collagen-Füllstoff mit einem Anteil von 0 bis ca. 40 Gew. % eingemischt ist.

13. Klebemittel- und Beschichtungsmasse nach Anspruch 1 zum Verbinden von Pflanzenmaterialien oder zum Beschichten von Pflanzenoberflächen, bei welcher sowohl das Additiv als auch der Füllstoff biochemisch mit der Pflanze, auf die die Masse angewendet wird, verträglich sind.

14. Klebemittelmasse nach Anspruch 1 zur Verbindung von Substraten, durch welche ein elektrischer Strom passieren soll, wobei die Masse zusätzlich elektrisch leitende Additive mit einem Anteil im Bereich von ca. 10 bis ca. 80 Gew. % enthält.

15. Verfahren zum Verbinden von zwei oder mehreren Substraten in einer Unterwasser-Umgebung, bei dem auf die zu verbindenden Oberflächen entweder unter Wasser oder vor dem Eintauchen in Wasser eine Klebemittelmasse gemäß Anspruch 1 aufgetragen wird.

16. Verfahren zum Beschichten einer Unterwasser-Oberfläche zum Verhindern des Wachstums von Mikroben und Pflanzen oder des Festsetzens von Meerestieren, wobei das Verfahren ein Aufbringen einer Masse entsprechend Anspruch 3 auf die Oberfläche umfaßt.

17. Verfahren zum Verhindern der Korrosion an einer Unterwasser-Oberfläche, wobei auf die Oberfläche eine Beschichtungsmasse entsprechend Anspruch 3 aufgebracht wird.

18. Verfahren zur Vorbereitung einer Oberfläche für eine nachfolgende Aufbringung einer Beschichtung oder eines Klebemittels, wobei das Verfahren das Aufbringen einer Masse gemäß Anspruch 1 auf die Oberfläche umfaßt.

19. Masse nach Anspruch 6 zur Aufbringung auf eine Oberfläche, auf der eine Adhäsion zum Zwecke der orthopädischen Instandsetzung oder Rekonstruktion gewünscht ist, zum Befestigen von Knochen, Sehnen, Bändern, Meniskus, Muskeln, eines jeden an einem jeden anderen oder zum Befestigen von eines jeden dieser Substrate an alloplastischen Materialien.

20. Masse nach Anspruch 19, bei welcher der bioverträgliche Füllstoff Collagen ist.

21. Masse nach Anspruch 19, bei welcher das Vernetzungsmittel Pilz-Tyrosinase ist.

22. Masse nach Anspruch 5 zur Auftragung auf eine Oberfläche, auf der die Anhaftung oder Reparatur für zahnärztliche Zwecke gewünscht ist, um Rückhalteteile, Brücken, Kronen oder Füllmaterialien an Zahnteilen

zu befestigen, zum Befestigen loser Zähne und zur Reparatur von zerbrochenen Zähnen.

23. Masse nach Anspruch 5 zur Aufbringung auf eine Oberfläche, auf der danach ein Donorgewebe oder alloplastisches Implantat befestigt wird zur Beschleunigung des Schließens von Wunden oder Einschnitten und zum Verhindern von bakterieller Verunreinigung in Weichteilen.

24. Masse nach Anspruch 5 zur Verwendung auf einer abzudichtenden Fläche zum Abdichten von Wund- oder Inzisionsverschlüssen und dazu benachbarten Bereiche, die durch zugehörige chirurgische Verschließvorrichtungen punktiert wurden, um ein Austreten von Flüssigkeiten hierdurch wesentlich zu vermindern.

25. Masse nach Anspruch 5 zur Verwendung auf einer zu behandelnden Oberfläche zur Behandlung von Wunden, Brüchen und Dislokationen bei Tieren.

26. Masse nach Anspruch 5 zur Verwendung als Klebemittel zum Kleben von prothetischen oder medizinischen Geräten, welche Medikamente, Medikationen, einzelne Zellen oder Zellagen oder elektronische Schaltkreise enthalten, die bei einem Patienten an einer vorgegebenen Stelle im Patienten implantiert werden sollen.

27. Masse nach Anspruch 10 zur Verwendung als ein Klebemittel, das auf Oberflächen aufzubringen ist, auf die danach alloplastische oder Donorgewebe zur Instandsetzung oder Befestigung von alloplastischen oder Donorgeweben in der Augenchirurgie befestigt werden sollen.

28. Masse nach Anspruch 10 zur Anwendung bei Augen-Inzisionen oder Punktierungen, die mit alloplastischem Material abzudichten sind.

29. Verfahren zur Behandlung von Pflanzen zum Zwecke der Instandsetzung von Verletzungen, der Rekonstruktion, des Wundverschlusses, der Pfropfung oder der Förderung der Hybridisierung oder genetischen Veränderung, welches ein Aufbringen einer Masse entsprechend Anspruch 13 auf Flächen beinhaltet, auf denen etwas angeheftet werden soll.

30. Verfahren zum Schutz von Pflanzen gegen den Befall von Pilzkrankheiten, welches das Aufbringen einer Beschichtungsmasse nach Anspruch 13 auf Oberflächenbereiche der Pflanze umfaßt, welche einem Pilzbefall ausgesetzt sind.

31. Verfahren zur Verbindung von zwei Substraten, durch welche ein elektrischer Strom zu leiten ist, welches das Verbinden der Substrate miteinander durch eine Klebemittelmasse gemäß Anspruch 14 umfaßt.

32. Verfahren zur Trennung von Schwermetallen und Verunreinigungen aus Fluiden, welches ein Aufbringen einer Beschichtungsmasse gemäß Anspruch 1 auf Nitrocellulosefilter oder auf Kunstharzperlen umfaßt, welche als Trägermatritzen in Ultrafiltrationsverfahren bzw. der Säulenchromatographie verwendet werden.

**Patentansprüche für folgende Vertragsstaaten: AT, ES, GR**

1. Verfahren zur Herstellung eines Klebemittels oder einer Beschichtung mittels der Herstellung einer Masse, die umfaßt:

(1) eine bioadhäsive polyphenolische Protein-Komponente mit einem Anteil von ca. 5 bis ca. 99 Gew. % einer Protein-Substanz, welche 1 bis ungefähr 1000 Einheiten der folgenden Decapeptid-Struktureinheit umfaßt:

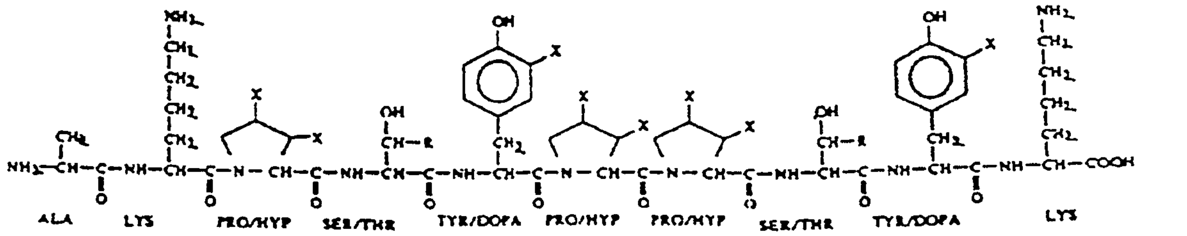

in welcher jedes X Wasserstoff oder eine Hydroxyl-Gruppe und jedes R Wasserstoff oder eine Methyl-Gruppe ist;

(2) ca. 0,1 bis ca. 40 Gew. % eines Vernetzungsmittels, welches die Vernetzung der Decapeptide fördert;

(3) eines oder mehrere Additive, welche die gewünschten Eigenschaften der Masse fördern, wobei die Additive mindestens eine oberflächenaktive Substanz umfassen und mit einem Anteil von 0 bis ca. 90 Gew. % enthalten sind; und

(4) einen mit dem Verwendungszweck der Masse verträglichen Füllstoff, wobei der Füllstoff mit einem Anteil von 0 bis ca. 50 Gew. % enthalten ist.

2. Verfahren nach Anspruch 1, bei welchem zum Herstellen eines wasserundurchlässigen Klebemittels

oder einer Beschichtung die bioadhäsive polyphenolische Protein-Komponente und das Vernetzungsmittel zusammen mit einem Anteil im Bereich von ca. 25 bis ca. 90 Gew. % der Masse verwendet werden.

3. Verfahren nach Anspruch 2 zur Herstellung einer Masse zur Verwendung in einer Unterwasser-Umgebung, bei dem:

(a) die bioadhäsive polyphenolische Protein-Komponente und das Vernetzungsmittel zusammen mit einem Anteil im Bereich von ca. 50 bis ca. 80 Gew. % verwendet werden; und

(b) eine oberflächenaktive Substanz, die als Netzmittel fungiert, bei den Additiven verwendet wird, mit einem Anteil im Bereich von ca. 20 bis ca. 40 Gew. % der Masse.

4. Verfahren nach Anspruch 3, bei welchem Natriumdodecylsulfat oder Natriumdodecylbenzolsulfonat als oberflächenaktive Substanz verwendet wird.

5. Verfahren nach Anspruch 1 zur Herstellung eines biomedizinisch verwendbaren Klebemittels oder Beschichtung, wobei ausschließlich solche Additive und Füllstoffe verwendet werden, welche biomedizinisch mit den Organen, auf die die Masse angewendet werden soll, verträglich sind.

6. Verfahren nach Anspruch 5 zur Herstellung eines Klebemittels, das bei der orthopädischen Instandsetzung, Rekonstruktion oder Prothetik verwendbar ist, bei welchem

(a) die bioadhäsive polyphenolische Komponente und das Vernetzungsmittel zusammen mit einem Anteil im Bereich von ca. 30 bis ca. 70 Gew. % verwendet werden;

(b) der Füllstoff mit einem Anteil im Bereich von ca. 30 bis ca. 60 Gew. % verwendet wird.

7. Verfahren nach Anspruch 6, bei welchem ein Füllstoff verwendet wird, der aus der Gruppe ausgewählt ist, die Collagen, Hyaluronsäure, Chondroitinsulfat, Elastin, Laminin, Casein, Hydroxyapatit oder eine ähnlich natürlich vorkommende, proteinhaltige, anorganische oder eine Mucopolysaccharid-Verbindung umfaßt.

8. Verfahren nach Anspruch 7, bei welchem Collagen als Füllstoff verwendet wird.

9. Verfahren nach Anspruch 7, bei welchem Pilz-Tyrosinase als Vernetzungsmittel verwendet wird.

10. Verfahren nach Anspruch 1 zur Herstellung eines Klebemittels zur Verwendung in der Augenchirurgie, bei welchem die bioadhäsive, polyphenolische Protein-Komponente und das Vernetzungsmittel zusammen mit einem Anteil variierend von ca. 70 bis ca. 100 Gew. % verwendet werden.

11. Verfahren nach Anspruch 10, bei welchem Pilz-Tyrosinase als Vernetzungsmittel verwendet wird.

12. Verfahren nach Anspruch 10 zur Herstellung eines Klebemittels, das für das Abdichten von Augen-Inzisionen und Perforationen mit alloplastischem Material verwendbar ist, bei welchem die bioadhäsive polyphenolische Protein-Komponente und das Vernetzungsmittel zusammen mit einem Anteil variierend von ca. 3 bis ca. 100 Gew. % verwendet werden und bei welchem ein Collagen-Füllstoff mit einem Anteil im Bereich von 0 bis ca. 40 Gew. % eingemischt wird.

13. Verwendung einer Klebemittel- und Beschichtungsmasse, die nach einem Verfahren gemäß Anspruch 1 hergestellt ist, zum Verbinden von Pflanzenmaterialien oder zum Beschichten von Pflanzenoberflächen, bei welchem sowohl das Additiv als auch der Füllstoff biochemisch mit der Pflanze, auf die die Masse angewendet wird, verträglich ist.

14. Verwendung einer Klebemittelmasse, die gemäß einem Verfahren nach Anspruch 1 hergestellt ist, zum Verbinden von Substraten, durch welche ein elektrischer Strom geleitet werden soll, wobei die Masse zusätzlich elektrisch leitende Additive mit einem Anteil im Bereich von ca. 10 bis ca. 80 Gew. % enthält.

15. Verfahren zur Verbindung von zwei oder mehreren Substraten in einer Unterwasser-Umgebung, welches das Anwenden einer Klebemittelmasse, die gemäß Anspruch 1 hergestellt ist, entweder unter Wasser oder vor dem Eintauchen in Wasser auf die zusammenzufügenden Oberflächen umfaßt.

16. Verfahren zum Beschichten einer Unterwasser-Oberfläche zur Verhinderung des Wachstums von Mikroben oder Pflanzen oder dem Anhaften von Meerestieren darauf, wobei das Verfahren den Schritt des Aufbringens einer Masse, die gemäß Anspruch 3 hergestellt ist, auf diese Oberfläche umfaßt.

17. Verfahren zur Verhinderung von Korrosion einer Unterwasser-Oberfläche, welches den Schritt umfaßt, daß eine Beschichtungsmasse nach Anspruch 3 auf die Oberfläche aufgetragen wird.

18. Verfahren zum Vorbereiten einer Oberfläche für eine nachfolgende Aufbringung einer Beschichtung oder eines Klebemittels, wobei das Verfahren den Schritt umfaßt, daß auf die Oberfläche eine gemäß Anspruch 1 hergestellte Masse aufgetragen wird.

19. Verfahren zur orthopädischen Instandsetzung oder Rekonstruktion zur Verbindung von Knochen, Sehnen, Bändern, Meniskus und Muskeln, jeweils miteinander oder eines jeden mit alloplastischem Material, wobei das Verfahren den Schritt umfaßt, daß auf eine Oberfläche, auf der etwas zu befestigen ist, eine Masse gemäß Anspruch 6 aufgetragen wird.

20. Verfahren nach Anspruch 19, bei dem die Masse als biokompatiblen Füllstoff Collagen enthält.

21. Verfahren nach Anspruch 19, bei dem die Masse als Vernetzungsmittel Pilz-Tyrosinase enthält.

22. Verfahren nach Anspruch 1 für die Herstellung von Klebemitteln zum Befestigen von Rückhalteteilen, Brücken, Kronen oder Füllstoffmaterialien an Zahnstrukturen, zum Sichern loser Zähne und zur Reparatur zerbrochener Zähne, bei welchem ausschließlich solche Additive und Füllstoffe verwendet werden, welche biomedizinisch mit den Oberflächen, mit denen das Klebemittel in Kontakt kommt, verträglich sind.

23. Verfahren nach Anspruch 1 zur Herstellung eines Klebemittels zum Befestigen von Donor-Gewebe oder alloplastischen Transplantaten zur Förderung des Wund- und Inzisionsverschlusses und zur Verhinderung einer bakteriellen Kontamination von Weichteilen, bei welchem ausschließlich solche Additive und Füllstoffe verwendet werden, welche biomedizinisch mit den Oberflächen, mit denen das Klebemittel in Kontakt kommt, verträglich sind.

24. Verfahren nach Anspruch 1 zur Herstellung einer dichtenden Masse zum Abdichten von Wund- oder Inzisionsverschlüssen und den hierzu benachbarten Bereichen, die durch zugehörige chirurgische Verschlußgeräte punktiert worden sind, um das Austreten von Flüssigkeit hieraus wesentlich zu vermindern, bei welchem ausschließlich solche Additive und Füllstoffe verwendet werden, welche biomedizinisch mit den Oberflächen, mit denen die Klebemittel in Kontakt kommen, verträglich sind.

25. Verfahren nach Anspruch 1 zur Herstellung einer Masse zum Behandeln von Wunden, Brüchen und Ausrenkungen bei Tieren, bei welchem ausschließlich solche Additive und Füllstoffe verwendet werden, welche biomedizinisch mit den Oberflächen, mit denen das Klebemittel in Kontakt kommt, verträglich sind.

26. Verfahren nach Anspruch 1 zur Herstellung eines Klebemittels für die Implantation einer Prothese oder eines medizinischen Gerätes, welches Arzneimittel, Medikationen, einzelne Zellen oder Zellagen enthält oder elektronische Schaltkreise in einen Patienten, bei welchem ausschließlich solche Additive und Füllstoffe verwendet werden, welche biomedizinisch mit den Oberflächen, mit denen das Klebemittel in Kontakt kommt, verträglich sind.

27. Verfahren der Augenchirurgie zur Reparatur oder dem Befestigen von alloplastischem oder Donor-Gewebe, welches den Schritt umfaßt, daß auf die Oberflächen, an denen befestigt werden soll, eine Klebemittelmasse entsprechend Anspruch 10 aufgetragen wird und daß das alloplastische oder Donor-Gewebe hierauf befestigt wird.

28. Verfahren zum Abdichten von Augen-Inzisionen oder Punktionen mit alloplastischem Material, welches den Schritt umfaßt, daß auf die zu behandelnde Oberfläche eine Masse entsprechend Anspruch 10 aufgetragen und das alloplastische Material hierauf befestigt wird.

29. Verfahren zur Behandlung von Pflanzen zum Zwecke der Instandsetzung von Verletzungen, der Rekonstruktion, des Wundverschlusses, des Transplantierens oder der Förderung der Hybridisierung oder genetischen Veränderung, wobei das Verfahren den Schritt umfaßt, daß auf Oberflächen, auf denen etwas anzuheften ist, eine Masse aufgetragen wird, die umfaßt:

(1) eine bioadhäsive polyphenolische Protein-Komponente mit ca. 5 bis ca. 99 Gew. % einer Protein-Substanz, welche eine bis ca. 1000 Einheiten der folgenden Decapeptid-Struktureinheit umfaßt:

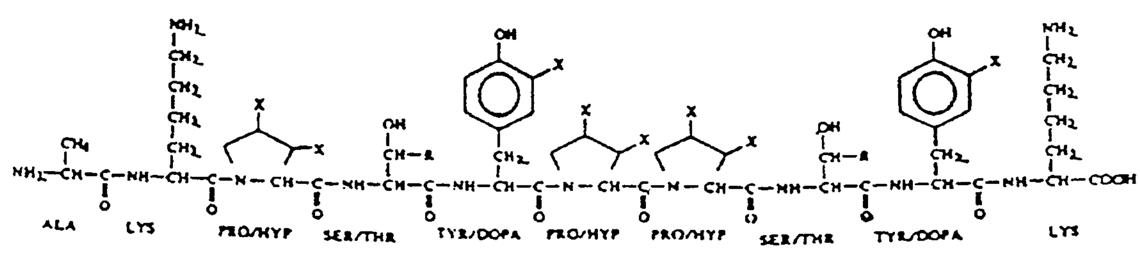

in welcher jedes X Wasserstoff oder eine Hydroxyl-Gruppe und jedes R Wasserstoff oder eine Methyl-Gruppe ist;

(2) ca. 0,1 bis ca. 40 Gew. % eines Vernetzungsmittels, welches das Vernetzen des Decapeptides fördert;

(3) eines oder mehrere Additive, die die gewünschten Eigenschaften der Masse bewirken, wobei die Additive mindestens eine oberflächenaktive Substanz umfassen und mit einem Anteil von 0 bis ca. 90 Gew. % enthalten sind; und

(4) einen Füllstoff, der mit der vorgesehenen Verwendung der Masse verträglich ist, wobei der Füllstoff mit einem Anteil von 0 bis ca. 50 Gew. % enthalten ist und bei welchem jedes Additiv und jeder Füllstoff biochemisch mit der Pflanze, auf die die Masse aufzutragen ist, verträglich ist.

30. Verfahren zum Schutz von Pflanzen gegen den Befall von Pilzkrankheiten, welches das Aufbringen einer Beschichtungsmasse auf Oberflächenbereiche der Pflanze umfaßt, die einem Pilzbefall ausgesetzt sind, wobei die Masse umfaßt:

(1) eine bioadhäsive polyphenolische Protein-Komponente mit ca. 5 bis ca. 99 Gew. % einer Protein-Substanz, welche eine bis ca. 1000 Einheiten der folgenden Decapeptid-Struktureinheit umfaßt:

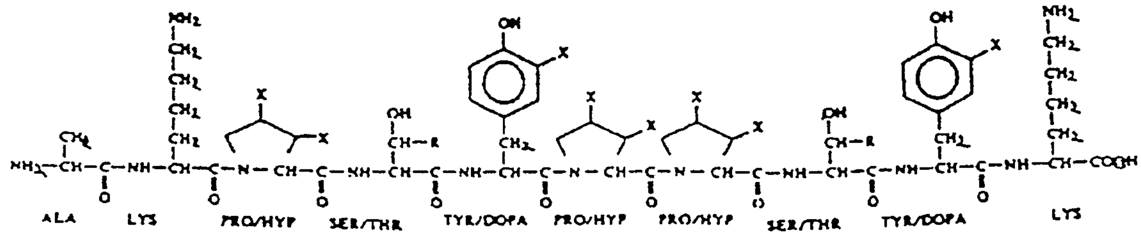

bei welcher jedes X Wasserstoff oder eine Hydroxyl-Gruppe und jedes R Wasserstoff oder eine Methyl-Gruppe ist;

(2) ca. 0,1 bis ca. 40 Gew. % eines Vernetzungsmittels, welches die Vernetzung der Decapeptide fördert;

(3) eines oder mehrere Additive, welche die gewünschten Eigenschaften der Masse fördern, wobei die Additive mindestens eine oberflächenaktive Substanz umfassen und mit einem Anteil von 0 bis ca. 90 Gew. % enthalten sind, und

(4) einen Füllstoff, welcher mit dem vorgesehenen Verwendungszweck der Masse kompatibel ist, wobei der Füllstoff mit einem Anteil von 0 bis ca. 50 Gew. % enthalten ist und bei welchem sowohl das Additiv als auch der Füllstoff biochemisch kompatibel mit der Pflanze, auf die die Masse anzuwenden ist.

31. Verfahren zum Verbinden zweier Substrate, durch welche ein elektrischer Strom zu leiten ist, welches das Zusammenfügen der Substrate miteinander mittels einer Klebemittelmasse umfaßt, welchletztere enthält:

(1) eine bioadhäsive polyphenolische Protein-Komponente mit ca. 5 bis ca. 99 Gew. % einer Protein-Substanz, welche eine bis 1000 Einheiten der folgenden Decapeptid-Struktureinheit umfaßt:

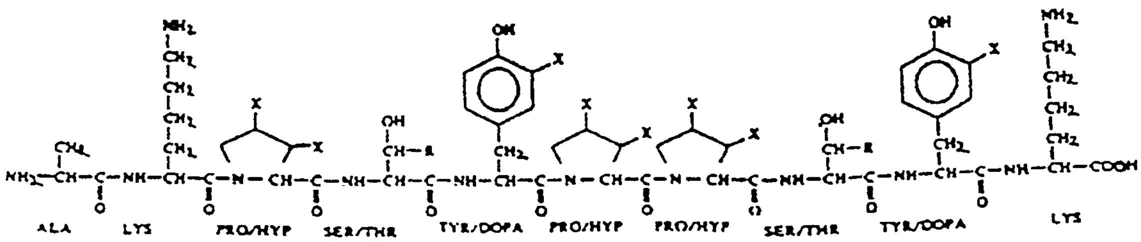

in welcher jedes X Wasserstoff oder eine Hydroxyl-Gruppe und jedes R Wasserstoff oder eine Methyl-Gruppe ist;

(2) ca. 0,1 bis ca. 40 Gew. % eines Vernetzungsmittels, welches die Vernetzung des Decapeptides fördert;

(3) ein oder mehrere Additive, welche die gewünschten Eigenschaften der Masse bewirken, wobei die Additive mindestens eine oberflächenaktive Substanz umfassen und mit einem Anteil von 0 bis 90 Gew. % enthalten sind; und

(4) ein Füllstoff, welcher mit dem vorgesehenen Verwendungszweck der Masse kompatibel ist, wobei der Füllstoff mit einer Menge von 0 bis ca. 50 Gew. % enthalten ist, wobei die Masse zusätzlich elektrisch leitende Additive mit einem Anteil im Bereich von ca. 10 bis ca. 80 Gew. % umfaßt.

32. Verfahren zur Trennung von Schwermetallen und Verunreinigungen aus Fluiden, welches die Anwendung einer Beschichtungsmasse auf Nitrocellulosefilter oder auf Harzperlen umfaßt, welche als Trägermatritzen in Ultrafiltrationsverfahren bzw. in der Säulenchromatographie verwendet werden, wobei die Masse umfaßt:

(1) eine bioadhäsive polyphenolische Protein-Komponente mit ca. 5 bis ca. 99 Gew. % einer Protein-Substanz, welche eine bis ca. 1000 Einheiten der folgenden Decapeptid-Struktureinheit umfaßt:

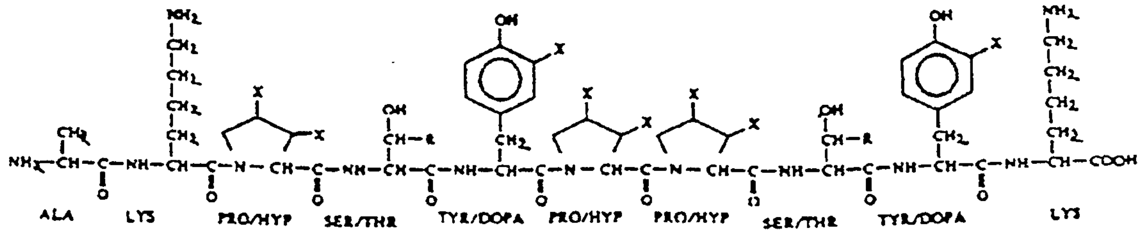

wobei jedes X Wasserstoff oder eine Hydroxyl-Gruppe und jedes R Wasserstoff oder eine Methyl-Gruppe ist;

(2) ca. 0,1 bis ca. 40 Gew. % eines Vernetzungsmittels, welches die Vernetzung der Decapeptide fördert;

(3) ein oder mehrere Additive, welche die gewünschten Eigenschaften der Masse fördern, wobei die Additive mindestens eine oberflächenaktive Substanz umfassen und mit einem Anteil von 0 bis ca. 90 Gew. % enthalten sind; und

(4) ein Füllstoff, der mit dem vorgesehenen Verwendungszweck der Masse verträglich ist, wobei der Füllstoff mit einem Anteil von 0 bis ca. 50 Gew. % enthalten ist.

## Revendications

### Revendications pour les Etats contractants suivants: BE, CH, DE, FR, GB, IT, LI, LU, NL, SE

1. Composition d'adhésif ou de revêtement comprenant :

(1) un composant protéine polyphénolique bioadhésive, contenant d'environ 5 à environ 99 % en poids d'une substance protéinée comprenant de 1 à environ 1 000 motifs de décapeptide récurrent suivant :

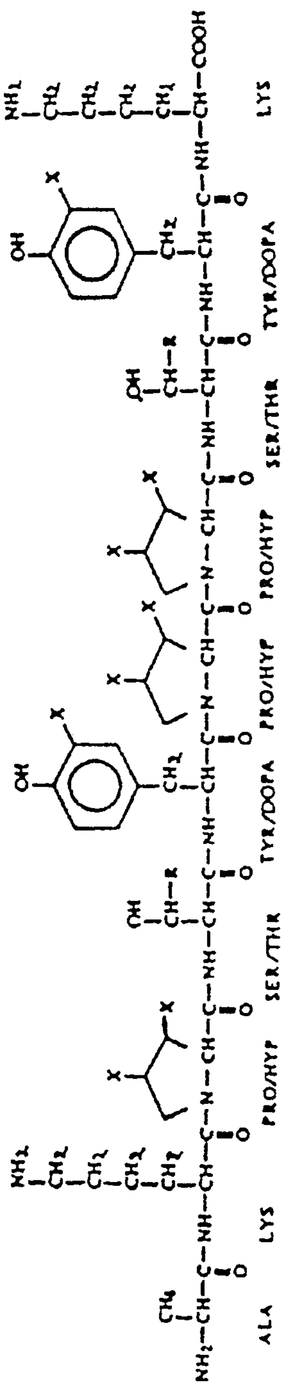

dans lequel chaque X est l'hydrogène ou un groupe hydroxyle et chaque R est l'hydrogène ou un groupe méthyle :

(2) d'environ 1,0 à environ 40 % en poids d'un agent réticulant qui stimule la réticulation du décapeptide ;

(3) un ou plusieurs additifs qui stimulent les propriétés désirées de la composition, lesdits additifs comprenant au moins un agent tensioactif et étant présents en une quantité comprise entre 0 % et environ 90 % en poids ; et

(4) une charge compatible avec l'usage prévu pour la composition, ladite charge étant présente en une quantité comprise entre 0 % et environ 50 % en poids.

2. Composition d'adhésif ou de revêtement imperméable à l'eau selon la revendication 1, dans laquelle le composant protéine polyphénolique bioadhésive et l'agent réticulant sont présents ensemble en une quantité comprise entre environ 25 et environ 90 % en poids de la composition.

3. Composition de revêtement imperméable à l'eau selon la revendication 2, à utiliser dans un environnement sous l'eau dans lequel :

(a) le composant protéine polyphénolique bioadhésive et l'agent réticulant sont présents ensemble en une quantité comprise entre environ 50 et environ 80 % en poids ; et

(b) les additifs comprennent un agent tensioactif agissant commme agent d'étalement, en une quantité comprise entre environ 20 et environ 40 % en poids de la composition.

4. Composition selon la revendication 3, dans laquelle l'agent tensioactif est du dodécylsulfate de sodium ou du dodécylbenzènesulfonate de sodium.

5. Composition adhésive selon la revendication 1, à usage biomédical, dans laquelle chaque additif et charge est biomédicalement compatible avec les organes sur lesquels la composition est appliquée.

6. Composition adhésive biomédicale selon la revendication 5, pour usage dans la réfection orthopédique, la restauration ou les prothèses, dans laquelle

(a) le composant polyphénolique bioadhésif et l'agent réticulant sont présents ensemble en une quantité comprise entre environ 30 et environ 70 % en poids ;

(b) la charge est présente en une quantité comprise entre environ 30 et environ 60 % en poids.

7. Composition adhésive biomédicale selon la revendication 6, dans laquelle la charge est sélectionnée dans le groupe comprenant le collagène, l'acide hyaluronique, le sulfate de chondroïtine, l'élastine, la laminine, la caséine, l'hydroxyapatite ou une susbtance protéinée, inorganique ou mucopolysaccharidique similaire naturelle.

8. Composition selon la revendication 7, dans laquelle la charge est du collagène.

9. Composition selon la revendication 7, dans laquelle l'agent réticulant est de la tyrosinase de champignon.

10. Composition adhésive selon la revendication 1, pour usage en chirurgie ophtalmologique, dans laquelle le composant protéine polyphénolique bioadhésive et l'agent réticulent sont présents ensemble en une quantité variant entre environ 70 et environ 100 % en poids.

11. Composition adhésive selon la revendication 10, dans laquelle l'agent réticulant est de la tyrosinase de champignon.

12. Composition adhésive selon la revendication 10, pour fermer les incisions et perforations ophtalmiques à l'aide de substances alloplastiques, dans lesquelles le composant protéine polyphénolique bioadhésive et l'agent réticulant sont présents ensemble en une quantité comprise entre environ 3 et environ 100 % en poids et une charge collagène est incorporée en quantités comprises entre 0 et environ 40 % en poids.

13. Composition d'adhésif et de revêtement selon la revendication 1, pour coller des substances végétales ou revêtir des substances végétales, dans laquelle chaque additif et charge est biocompatible avec la plante sur laquelle la composition est appliquée.

14. Composition adhésive selon la revendication 1 pour réunir des substrats à travers lesquels on fera passer un courant électrique, ladite composition contenant additionnellement des additifs conducteurs de l'électricité en une quantité comprise entre environ 10 et environ 80 % en poids.

15. Procédé d'adhésion de deux ou plusieurs substrats dans un environnement sous l'eau, comprenant l'application aux surfaces adhérentes soit sous l'eau, soit avant l'immersion dans l'eau, d'une composition adhésive selon la revendication 1.

16. Procédé de revêtement d'une surface sous l'eau, de façon à prévenir la croissance de microbes et de plantes sur celle-ci, ou la fixation à celle-ci d'animaux marins, ledit procédé comprenant l'application à ladite surface d'une composition selon la revendication 3.

17. Procédé de prévention de la corrosion d'une surface sous l'eau, comprenant l'application à ladite surface d'une composition de revêtement selon la revendication 3.

18. Procédé de traitement de fond d'une surface en vue de l'application subséquente d'un revêtement ou d'un adhésif, lequel procédé comprend l'application à ladite surface d'une composition selon la revendication

1.

19. Composition selon la revendication 6 pour application sur une surface sur laquelle on recherche l'adhérence pour usage dans la réfection et la restauration orthopédiques, pour coller os, tendons, ligaments, ménisques, muscles les uns aux autres ou coller un quelconque de ces éléments à des substances alloplastiques.

20. Composition selon la revendication 19, dans laquelle la charge biocompatible est du collagène.

21. Composition selon la revendication 19, dans laquelle l'agent réticulant est de la tyrosinase de champignon.

22. Composition selon la revendication 5, pour application à une surface que laquelle l'adhérence ou la réparation est recherchée, pour usage en dentisterie pour coller appareils, bridges, couronnes ou plombages à la structure de la dent, pour fixer les dents mobiles et réparer les dents cassées.

23. Composition selon la revendication 5, pour application sur une surface sur laquelle on fixera ensuite le tissu de donneur ou les greffes alloplastiques, pour activer la fermeture des plaies et des incisions et prévenir les contaminations bactériennes dans les tissus mous.

24. Composition selon la revendication 5, pour application à un site à refermer, pour refermer les sutures des plaies ou des incisions et les régions proches qui ont été perforées par des dispositifs de fermeture chirurgicale associés, pour réduire sensiblement les suintements de liquides.

25. Composition selon la revendication 5, pour application à une surface à traiter, pour traiter les plaies, les fractures et les luxations chez les animaux.

26. Composition selon la revendication 5, pour usage comme adhésif, pour coller une prothèse ou un dispositif médical contenant des médicaments, des cellules simples ou des feuilles de cellules ou des circuits électroniques à implanter chez un malade au site désiré chez le malade

27. Composition selon la revendication 10, pour usage comme adhésif à appliquer à des surfaces sur lesquelles on fixera ensuite un tissu alloplastique ou le tissu d'un donneur pour réparer ou fixer le tissu alloplastique ou le tissu du donneur en chirurgie ophtalmique.

28. Composition selon la revendication 10, pour application à des incisions ou perforations ophtalmiques à obturer avec une substance alloplastique.

29. Procédé de traitement de plantes à des fins de réparation de lésions, de restauration, de fermeture des plaies, de greffage ou de stimulation de l'hybridation ou de modifications génétiques, comprenant l'application à des surfaces qui seront collées, d'une composition selon la revendication 13.

30. Procédé de protection des plantes contre l'attaque des maladies fongiques, comprenant l'application aux surfaces de la plante soumises à l'attaque fongique d'une composition de revêtement selon la revendication 13.

31. Procédé de réunion de deux substrats à travers lesquels on fera passer un courant électrique, comprenant l'adhésion des substrats l'un à l'autre à l'aide d'une composition adhésive selon la revendication 14.

32. Procédé de séparation de métaux lourds et de contaminants de liquides, comprenant l'application à un filtre en nitrocellulose ou à des billes en résine utilisées respectivement comme matrices supports dans des techniques d'ultrafiltration et de chromatographie sur colonne, d'une composition de revêtement selon la revendication 1.

## Revendications pour les Etats contractants suivants: AT, ES, GR

1. Procédé de fabrication d'un adhésif ou d'un revêtement par production d'une composition comprenant :
(1) un composant protéine polyphénolique bioadhésive, contenant d'environ 5 à environ 99 % en poids d'une substance protéinée comprenant de 1 à environ 1 000 motifs de décapeptide récurrent suivant :

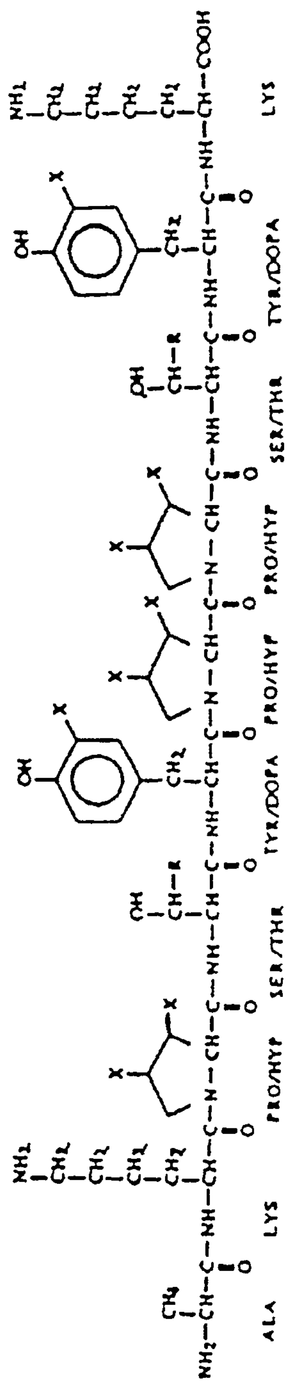

dans lequel chaque X est l'hydrogène ou un groupe hydroxyle et chaque R est l'hydrogène ou un groupe méthyle :

(2) d'environ 0,1 à environ 40 % en poids d'un agent réticulant qui stimule la réticulation du décapeptide ;

(3) un ou plusieurs additifs qui stimulent les propriétés désirées de la composition, lesdits additifs comprenant au moins un agent tensioactif et étant présents en une quantité comprise entre 0 % et environ 90 % en poids ; et

(4) une charge compatible avec l'usage prévu pour la composition, ladite charge étant présente en une quantité comprise entre 0 % et environ 50 % en poids.

2. Procédé selon la revendication 1 dans lequel, pour fournir un adhésif ou un revêtement imperméable à l'eau, le composant protéine polyphénolique bioadhésive et l'agent réticulant ensemble sont utilisés en une quantité comprise entre environ 25 et environ 90 % en poids de la composition.

3. Procédé selon la revendication 2 pour fournir une composition à utiliser dans un environnement sous l'eau dans lequel :

(a) le composant protéine polyphénolique bioadhésive et l'agent réticulant ensemble sont utilisés en une quantité comprise entre environ 50 et environ 80 % en poids ; et

(b) un agent tensioactif agissant comme agent d'étalement est utilisé dans les additifs en une quantité comprise entre environ 20 et environ 40 % en poids de la composition.

4. Procédé selon la revendication 3, dans lequel du dodécylsulfate de sodium ou du dodécylbenzènesulfonate de sodium est utilisé comme agent tensioactif.

5. Procédé selon la revendication 1 de fabrication d'un adhésif ou d'un revêtement biomédicalement utilisable dans lequel sont utilisés exclusivement des additifs et des charges biomédicalement compatibles avec les organes sur lesquels la composition sera appliquée.

6. Procédé selon la revendication 5 de fabrication d'un adhésif utilisable dans la réfection orthopédique, la restauration ou les prothèses, dans lequel :

(a) le composant polyphénolique bioadhésif et l'agent réticulant ensemble sont utilisés en une quantité comprise entre environ 30 et environ 70 % en poids ;

(b) la charge es utilisée en une quantité comprise entre environ 30 et environ 60 % en poids.

7. Procédé selon la revendication 6, dans lequel est utilisée une charge sélectionnée dans le groupe comprenant le collagène, l'acide hyaluronique, le sulfate de chondroïtine, l'élastine, la laminine, la caséine, l'hydroxyapatite ou une susbtance protéinée, inorganique ou mucopolysaccharidique similaire naturelle.

8. Procédé selon la revendication 7, dans lequel du collagène est utilisé comme charge.

9. Procédé selon la revendication 7, dans lequel de la tyrosinase de champignon est utilisée comme agent réticulant.

10. Procédé selon la revendication 1 de fabrication d'un adhésif pour usage en chirurgie ophtalmologique, dans lequel le composant protéine polyphénolique bioadhésive et l'agent réticulant ensemble sont utilisés en une quantité variant entre environ 70 et environ 100 % en poids.

11. Procédé selon la revendication 10, dans lequel de la tyrosinase de champignon est utilisée comme agent réticulant.

12. Procédé selon la revendication 10 de fabrication d'un adhésif utilisable pour fermer les incisions et perforations ophtalmiques à l'aide de substances alloplastiques, dans lequel le composant protéine polyphénolique bioadhésive et l'agent réticulant ensemble sont utilisés en une quantité variant entre environ 3 et environ 100 °% en poids et une charge collagène est incorporée en quantités comprises entre 0 et environ 40 % en poids.

13. Emploi d'une composition d'adhésif et de revêtement produite selon le procédé défini dans la revendication 1 pour coller des substances végétales ou revêtir des substances végétales, dans laquelle chaque additif et charge est biochimiquement compatible avec la plante sur laquelle la composition est appliquée.

14. Emploi d'une composition adhésive produite selon le procédé défini dans la revendication 1 pour réunir des substrats à travers lesquels on fera passer un courant électrique, ladite composition contenant additionnellement des additifs conducteurs de l'électricité en une quantité comprise entre environ 10 et environ 80 % en poids.

15. Procédé d'adhésion de deux ou plusieurs substrats dans un environnement sous l'eau, comprenant l'application aux surfaces adhérentes, soit sous l'eau, soit avant l'immersion dans l'eau, d'une composition adhésive produite selon la revendication 1.

16. Procédé de revêtement d'une surface sous l'eau, de façon à prévenir la croissance de microbes ou de plantes sur celle-ci, ou la fixation à celle-ci d'animaux marins, ledit procédé comprenant l'application à ladite surface d'une composition produite selon la revendication 3.

17. Procédé de prévention de la corrosion d'une surface sous l'eau, comprenant l'application à ladite surface d'une composition de revêtement produite selon la revendication 3.

18. Procédé de traitement de fond d'une surface en vue de l'application subséquente d'un revêtement ou d'un adhésif, lequel procédé comprend l'application à ladite surface d'une composition produite selon la revendication 1.

19. Dans la réfection et la restauration orthopédiques, procédé pour coller os, tendons, ligaments, ménisques, muscles les uns aux autres ou coller un quelconque de ces éléments à des substances alloplastiques, lequel procédé comprend l'application, sur une surface sur laquelle on recherche l'adhérence, d'une composition selon la revendication 6.

20. Procédé selon la revendication 19, dans lequel, dans la composition, la charge biocompatible est du collagène.

21. Procédé selon la revendication 19, dans lequel, dans la composition, l'agent réticulant est de la tyrosinase de champignon.

22. Procédé selon la revendication 1 de fabrication d'un adhésif pour coller appareils, bridges, couronnes ou plombages à la dent, pour fixer les dents mobiles et réparer les dents cassées, dans lequel sont utilisés exclusivement des additifs et des charges biomédicalement compatibles avec les surfaces avec lesquelles l'adhésif vient en contact.

23. Procédé selon la revendication 1 de fabrication d'un adhésif pour fixer un tissu de donneur ou des greffes alloplastiques pour activer la fermeture des plaies et des incisions et prévenir les contaminations bactériennes dans les tissus mous, dans lequel sont utilisés exclusivement des additifs et des charges biomédicalement compatibles avec les surfaces avec lesquelles l'adhésif vient en contact.

24. Procédé selon la revendication 1 de fabrication d'une composition de fermeture pour refermer les sutures des plaies ou des incisions et les régions proches qui ont été perforées par des dispositifs de fermeture chirurgicale associés, pour réduire sensiblement les suintements de liquides, dans lequel sont utilisés exclusivement des additifs et des charges biomédicalement compatibles avec les surfaces avec lesquelles l'adhésif vient en contact.

25. Procédé selon la revendication 1 de fabrication d'une composition pour traiter les plaies, les fractures et les luxations chez les animaux, dans lequel sont utilisés exclusivement des additifs et des charges biomédicalement compatibles avec les surfaces avec lesquelles l'adhésif vient en contact.

26. Procédé selon la revendication 1 de fabrication d'un adhésif pour implanter chez un malade une prothèse ou un dispositif médical contenant des médicaments, des cellules simples ou des feuilles de cellules ou des circuits électroniques, dans lequel sont utilisés exclusivement des additifs et des charges biomédicalement compatibles avec les surfaces avec lesquelles l'adhésif vient en contact.

27. En chirurgie ophtalmologique, procédé de réparation ou de fixation d'un tissu alloplastique ou du tissu d'un donneur, comprenant l'application sur les surfaces à coller d'une composition d'adhésif selon la revendication 10 et la fixation à celles-ci dudit tissu alloplastique ou tissu de donneur.

28. Procédé de fermeture des incisions ou perforations ophtalmiques avec une substance alloplastique, comprenant l'application sur la surface à traiter d'une composition selon la revendication 10 et la fixation à celle-ci de ladite substance alloplastique.

29. Procédé de traitement de plantes à des fins de réparation de lésions, de restauration, de fermeture des plaies, de greffage ou de stimulation de l'hybridation ou de modifications génétiques, comprenant l'application à des surfaces qui seront collées, d'une composition comprenant :

(1) un composant protéine polyphénolique bioadhésive, contenant d'environ 5 à environ 99 % en poids d'une substance protéinée comprenant de 1 à environ 1 000 motifs de décapeptide récurrent suivant :

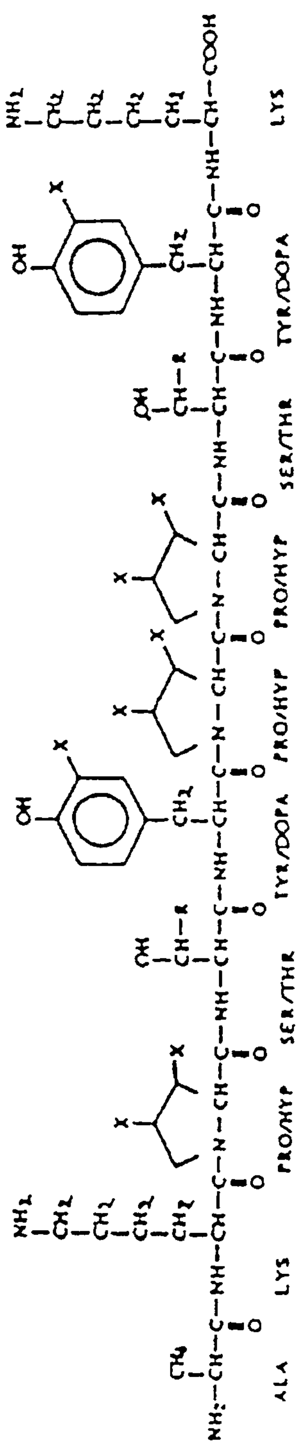

dans lequel chaque X est l'hydrogène ou un groupe hydroxyle et chaque R est l'hydrogène ou un groupe méthyle :

(2) d'environ 0,1 à environ 40 % en poids d'un agent réticulant qui stimule la réticulation du décapeptide ;

(3) un ou plusieurs additifs qui stimulent les propriétés désirées de la composition, lesdits additifs comprenant au moins un agent tensioactif et étant présents en une quantité comprise entre 0 % et environ 90 % en poids ; et

(4) une charge compatible avec l'usage prévu pour la composition, ladite charge étant présente en une quantité comprise entre 0 % et environ 50 % en poids, et dans lequel chaque additif et charge est biochimiquement compatible avec la plante sur laquelle la composition est appliquée.

30. Procédé de protection des plantes contre l'attaque des maladies fongiques, comprenant l'application aux surfaces de la plante soumises à l'attaque fongique d'une composition de revêtement comprenant :

(1) un composant protéine polyphénolique bioadhésive, contenant d'environ 5 à environ 99 % en poids d'une substance protéinée comprenant de 1 à environ 1 000 motifs de décapeptide récurrent suivant :

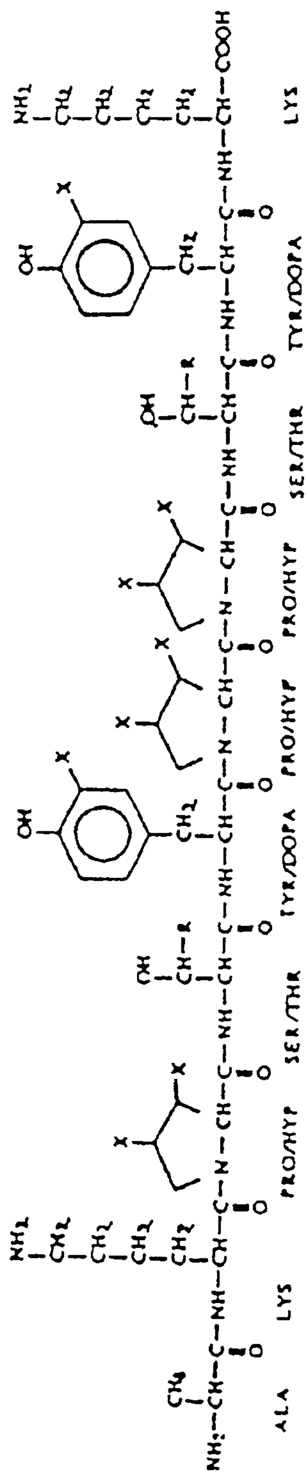

dans lequel chaque X est l'hydrogène ou un groupe hydroxyle et chaque R est l'hydrogène ou un groupe méthyle :

(2) d'environ 0,1 à environ 40 % en poids d'un agent réticulant qui stimule la réticulation du décapeptide ;

(3) un ou plusieurs additifs qui stimulent les propriétés désirées de la composition, lesdits additifs comprenant au moins un agent tensioactif et étant présents en une quantité comprise entre 0 % et environ 90 % en poids ; et

(4) une charge compatible avec l'usage prévu pour la composition, ladite charge étant présente en une quantité comprise entre 0 % et environ 50 % en poids, et dans lequel chaque additif et charge est biochimiquement compatible avec la plante sur laquelle la composition est appliquée.

31. Procédé de réunion de deux substrats à travers lesquels on fera passer un courant électrique, comprenant l'adhésion des substrats l'un à l'autre à l'aide d'une composition adhésive comprenant :

(1) un composant protéine polyphénolique bioadhésive, contenant d'environ 5 à environ 99 % en poids d'une substance protéinée comprenant de 1 à environ 1 000 motifs de décapeptide récurrent suivant:

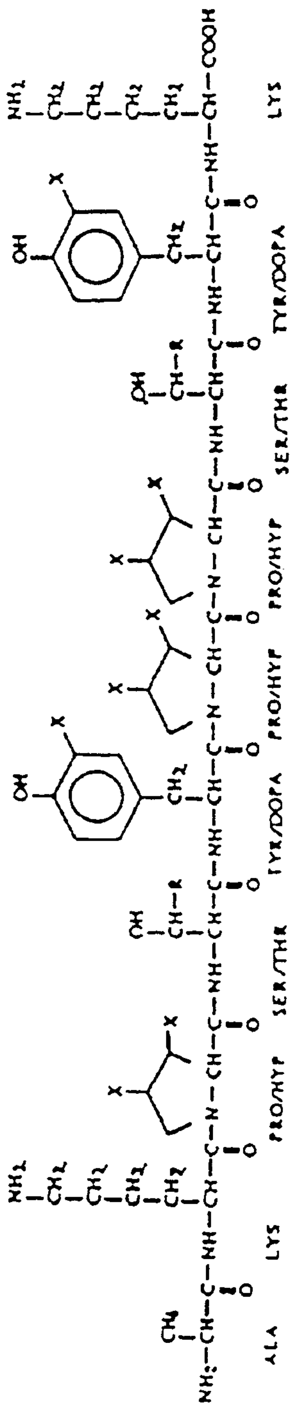
ALA
LYS
PRO/HYP
SER/THR
TYR/DOPA
PRO/HYP
PRO/HYP
SER/THR
TYR/DOPA
LYS

dans lequel chaque X est l'hydrogène ou un groupe hydroxyle et chaque R est l'hydrogène ou un groupe méthyle :

(2) d'environ 0,1 à environ 40 % en poids d'un agent réticulant qui stimule la réticulation du décapeptide ;

(3) un ou plusieurs additifs qui stimulent les propriétés désirées de la composition, lesdits additifs comprenant au moins un agent tensioactif et étant présents en une quantité comprise entre 0 % et environ 90 % en poids ; et

(4) une charge compatible avec l'usage prévu pour la composition, ladite charge étant présente en une quantité comprise entre 0 % et environ 50 % en poids, ladite composition contenant additionnellement des additifs conducteurs de l'électricité en une quantité comprise entre environ 10 et environ 80 % en poids.

32. Procédé de séparation de métaux lourds et de contaminants de liquides, comprenant l'application à un filtre en nitrocellulose ou à des billes en résine utilisés respectivement comme matrices supports dans des techniques d'ultrafiltration et de chromatographie sur colonne, d'une composition de revêtement comprenant :

(1) un composant protéine polyphénolique bioadhésive, contenant d'environ 5 à environ 99 % en poids d'une substance protéinée comprenant de 1 à environ 1 000 motifs de décapeptide récurrent suivant:

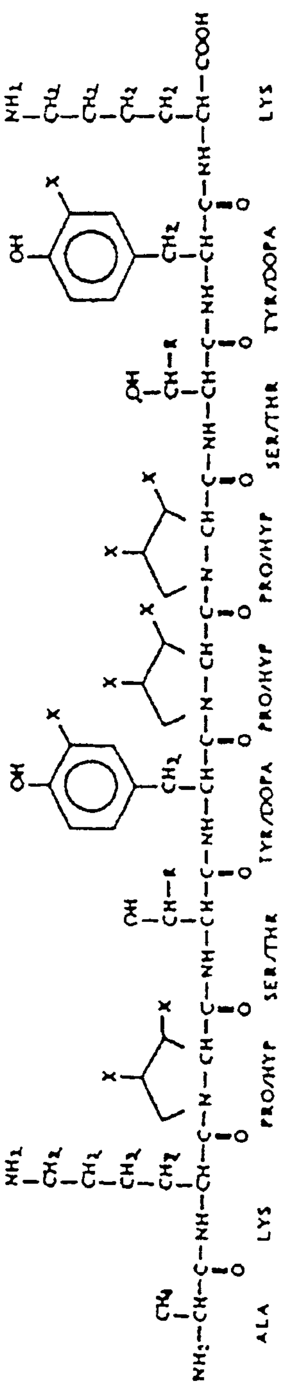

dans lequel chaque X est l'hydrogène ou un groupe hydroxyle et chaque R est l'hydrogène ou un groupe méthyle :
(2) d'environ 0,1 à environ 40 % en poids d'un agent réticulant qui stimule la réticulation du décapeptide ;
(3) un ou plusieurs additifs qui stimulent les propriétés désirées de la composition, lesdits additifs comprenant au moins un agent tensioactif et étant présents en une quantité comprise entre 0 % et environ 90 % en poids ; et
(4) une charge compatible avec l'usage prévu pour la composition, ladite charge étant présente en une quantité comprise entre 0 % et environ 50 % en poids.